# EUROPÄISCHE PATENTSCHRIFT

(11) **EP 3 709 869 B1**
(45) Veröffentlichungstag und Bekanntmachung des Hinweises auf die Patenterteilung: **06.07.2022**
(21) Anmeldenummer: 18832976.7
(22) Anmeldetag: 12.11.2018
(51) Int. Cl.: A61B 5/00, A61B 10/00, A61B 5/01, A61B 5/20, G01N 33/483

(54) **VORRICHTUNG ZUR VOR-ORT-ANALYSE VON EXKREMENTEN UND VERFAHREN ZUM BETREIBEN EINER DERARTIGEN VORRICHTUNG**
DEVICE FOR THE ON-SITE ANALYSIS OF EXCREMENTS AND METHOD FOR OPERATING SUCH A DEVICE
DISPOSITIF D'ANALYSE IN SITU D'EXCRÉMENTS ET PROCÉDÉ DE COMMANDE D'UN TEL DISPOSITIF

(30) Priorität: 14.11.2017 DE 102017010519
(43) Veröffentlichungstag der Anmeldung: 23.09.2020
(73) Patentinhaber: MEDIPEE GMBH, 47445 Moers (DE)
(72) Erfinder: PROKOPP, Thomas, 53125 Bonn (DE); BANDI, Paul, 47475 Kamp-Lintfort (DE); KRUISSEKBRINK, Eric, 7102 JH Winterswijk (NL); WILLEMS, Frank, 47441 Moers (DE)
(74) Vertreter: FARAGO Patentanwaltsgesellschaft mbH
(86) Internationale Anmeldenummer: PCT/DE2018/000328
(87) Internationale Veröffentlichungsnummer: WO 2019/096340

(56) Entgegenhaltungen:
- WO-A1-2017/021452
- DE-A1-102006 029 987
- DE-T2- 69 025 677
- JP-A- H02 309 930
- US-A1- 2003 211 634
- US-A1- 2007 122 914

## Beschreibung

Die Erfindung betrifft einerseits eine Vorrichtung zur Vor-Ort-Analyse von Exkrementen , wie in Anspruch 1 definiert.

Die Erfindung betrifft auch ein Verfahren zum Betreiben einer Vorrichtung zur Vor-Ort-Analyse von Exkrementen, wie in Anspruch 10 definiert.

Die Erfindung betrifft des Weiteren vorzugsweise eine Anordnung bestehend aus einer Toilette, einem

Urinal oder dergleichen und aus einer Vorrichtung zur Vor-Ort-Analyse von Exkrementen.

Die Erfindung betrifft ebenso vorzugsweise eine oben genannte Vorrichtung zur Vor-Ort-Analyse in Verbindung mit einem Probenträger für eine Vor-Ort-Analyse von Exkrementen.

Es ist bekannt, dass mittels Analysen von Urin- und/oder Stuhlproben zumindest erste Erkenntnisse zu einem Gesundheitszustand eines Organismus, insbesondere eines Menschen, ermittelt werden können. Um hierbei jedoch hinreichend aussagekräftige Analyseinformationen zu erhalten, ist es zumeist erforderlich, etwa an einem WC genommene Urin- und/oder Stuhlproben sicher in ein Transportgefäß zu verbringen, um dieses Gefäß mit der darin befindlichen Probe an ein Analyselabor zu senden. Dies ist nicht nur umständlich, sondern kann zeitlich auch länger dauern, bevor entsprechende Analyseergebnisse vorliegen. Besonders nachteilig ist es hierbei jedoch, dass ein engmaschiges Monitoring im Sinne von kontinuierlichen Vorsorgeuntersuchungen wegen des nicht unerheblichen Aufwands in der Regel ausbleibt. Insofern ist es wünschenswert, eine Methode zu schaffen, welche die Analyse von Exkrementen erleichtert.

Aus der WO 2017/021452 A1 ist insbesondere eine Vorrichtung zur mobilen Analyse von Exkrementen in einer Toilette bekannt, so dass eine tägliche Analyse von Urin- und/oder Stuhlproben vor Ort, das heißt sogleich an dem Ort der Probenentnahme, vorgenommen werden kann. Eine Vorrichtung gemäss der Präambel von Anspruch 1 ist von der DE 690 25 677 T2 bekannt.

Der Erfindung liegt die Aufgabe zugrunde, insbesondere eine gattungsgemäße Vorrichtung zum Analysieren von Exkrementen, speziell von menschlichen Exkrementen, weiterzuentwickeln, so dass die Vorrichtung zuverlässiger arbeitet.

Die Aufgabe der Erfindung wird nach einem ersten Aspekt der Erfindung von einer Vorrichtung zur Vor-Ort-Analyse von Exkrementen gemäss Anspruch 1 gelöst.

Dadurch, dass das Führungsteil einen Kontaminationsbereich sowie einen Reinbereich aufweist, kann die Vorrichtung zuverlässiger betrieben werden.

Insbesondere kann hierbei die Gefahr zur Gänze ausgeschlossen oder zumindest signifikant verringert werden, dass Exkremente, insbesondere Exkremente eines anderen Benutzers, unbeabsichtigt einen neuen Probenträger kontaminieren, während dieser entlang des Führungsteils zur Probenentnahme bewegt wird, nämlich dann wenn das bewegliche Armelement mit dem neuen Probenträger für eine neue Probenentnahme entlang der Führungsbahn axial verschoben wird. Hierdurch wiederum sinkt die Gefahr, dass Analyseergebnisse verfälscht werden.

Insofern ist ein ständiges mobiles Monitoring mittels Urin- und/oder Stuhlproben noch einfacher und zuverlässiger durchführbar.

Im Sinne der Erfindung ist das bewegliche Armelement gegenüber dem Führungsteil mit einer axialen Verschieberichtung axial verschieblich gelagert, wobei sich die axiale Verschieberichtung entlang der Längserstreckung des Führungsteils orientiert.

Genauer gesagt, ist das bewegliche Armelement zum Entnehmen einer neuen Probe an Exkrementen gemeinsam mit einem neuen, also unbenutzten, Probenträger in axialer Ausfahrrichtung zumindest teilweise aus dem Führungsteil axial ausfahrbar.

Ferner ist das bewegliche Armelement gemeinsam mit dem mit Urin oder Stuhl benetzten, also benutzten Probenträger in axialer Einfahrrichtung wieder das Führungsteil axial einfahrbar.

Insofern bezieht sich die Begrifflichkeit "axial" auf die eigentliche Verschiebe- bzw. Bewegungsrichtung des beweglichen Armelements bzw. des Probenträgers.

Mit der Begrifflichkeit "Entnahmeeinrichtung" ist im Sinne der Erfindung jegliche Einrichtung erfasst, mittels welcher eine Probe an Exkrement entnommen und der Vorrichtung zur Analyse zugeführt werden kann.

Die Begrifflichkeit "Beschickungseinrichtung" beschreibt vorliegend eine Einrichtung, mittels welche ein neuer Probenträger an das bewegliche Armelement übergeben werden kann.

Der Begriff "Analyseeinrichtung" erfasst vorliegend sämtliche Einrichtungen, welche dazu geeignet sind, eine Probe an Exkrement sensorisch zu erfassen und zu analysieren.

Hierzu umfasst die Analyseeinrichtung auch wenigstens einen Sensor, vorzugsweise eine ganze Sensoreinheit mit einer Vielzahl an gleichen oder bevorzugt unterschiedlichen Sensoren.

Der Begriff "Kontaminationsbereich" beschreibt im Sinne der Erfindung denjenigen Bereich des Führungsteils, in welchem das bewegliche Armelement geführt ist. Insofern befindet sich auch die Führungsbahn des Führungsteils in diesem Kontaminationsbereich. Hierbei kann nicht vollständig ausgeschlossen werden, dass das bewegliche Armelement mit Exkrement in Kontakt kommt.

Dieser Kontaminationsbereich erstreckt sich im Wesentlichen von einer an dem freien Ende des Führungsteils angeordneten Führungsbahnöffnung und einem Übergabebereich, in welchem ein neuer Probenträger von der Beschickungseinrichtung an einen an dem Armelement befindlichen Halter übergeben wird.

Der Begriff "Reinbereich" beschreibt vorliegend einen Bereich des Führungsteils, in welchem weder das bewegliche Armelement noch ein benutzter Probenträger hin gelangt.

Dieser Reinbereich beschreibt insbesondere einen Streckenbereich an dem Führungsteil zwischen einer an dem freien Ende des Führungsteils angeordneten Sensoreinheit und dem Übergabebereich, in welchem ein neuer Probenträger von der Beschickungseinrichtung an einen an dem Armelement befindlichen Halter übergeben wird.

Mit dem freien Ende des Führungsteils ist dasjenige Ende des Führungsteils bezeichnet, aus welchem das bewegliche Armelement für eine Probenentnahme aus dem Führungsteil herausfährt. An diesem freien Ende sitzt auch die Sensoreinheit.

Der Begriff "Sensoreinheit" beschreibt im Sinne der Erfindung eine Einrichtung, welche zumindest im Wesentlichen wenigstens einen Sensor, bevorzugt mehrere Sensoren umfasst, welche eine an dem Probenträger anhaftende Probe für eine Analyse sensorisch erfassen.

Vorliegend kann eine derartige Sensoreinheit unterschiedlichst konstruiert sein, um einen Probenträger gegenüber der Sensoreinheit zumindest temporär platzieren zu können und/oder um entsprechend den gestellten Anforderungen etwa an dem Führungsteil angeordnet bzw. ausgebildet zu sein.

Es versteht sich, dass hierzu unterschiedliche Sensoren vorgesehen sein können.

Beispielsweise umfasst die Sensoreinheit einen Farbsensor zum Detektieren eines Farbumschlags bezüglich des Probenträgers, wodurch bereits eine erste Analyse im Sinne der Erfindung durchführbar ist.

Weitere Daten der Sensoreinheit können kumulativ oder alternativ an eine Analyseeinheit gesendet werden, welche beispielsweise in dem Gehäuse der Vorrichtung und/oder aber auch an dem Führungsteil der Vorrichtung sitzen kann.

Ferner ist es möglich, dass kumulativ oder alternativ Daten von der Sensoreinheit und/oder von der Analyseeinheit an eine externe Analyseeinheit übermittelt werden. Gedacht sei hierbei nur zum Beispiel an eine auf einem Smartphone oder dergleichen laufende Datenverarbeitung- und/oder Analyseapplikation.

Die Übermittelung kann hierbei kabellos oder auch kabelgebunden erfolgen, wobei die Übermittelung zwischen der Vorrichtung und einer externen Datenverarbeitung- und/oder Analyseapplikation oder ähnlichem vorzugsweise kabellos erfolgt.

Vorteilhafterweise umfasst die Analyseeinrichtung, insbesondere die Sensoreinheit und/oder eine zusätzliche Analyseeinheit, für die Analyse einen geeigneten Microcontroller oder dergleichen, mittels welchem in der einfachsten Ausführung zum Beispiel ein Farbumschlag am Probenträger analysiert werden kann.

Es ist hierbei denkbar, dass zumindest einige der Sensoren je nach zu analysierendem Exkrement ab- oder zugeschaltet werden können, wodurch die Vorrichtung energetisch effizienter betrieben werden kann.

Das "bewegliches Armelement" kann konstruktiv unterschiedlichst ausgebildet sein, beispielsweise als teleskopierbares Armelement, mit ineinander einfahrbaren Armsegmenten oder dergleichen.

Bevorzugt umfasst das vorliegende Armelement jedoch einen auf- bzw. abrollbaren Materialstreifen auf, welcher zum Ausfahren des Armelements von einem Coil abgerollt und zum Einfahren des Armelements wieder auf das Coil aufgerollt werden kann.

Der Begriff "Probenträger" beschreibt ein Materialstück, welches zur Aufnahme von Exkrement geeignet ist, wie beispielsweise ein Indikatorstreifen oder -blättchen, oder dergleichen.

An dieser Stelle sei bereits erwähnt, dass ein solcher Probenträger vielfältig ausgestaltet sein kann, um im Sinne der vorliegenden Erfindung vorteilhaft eingesetzt werden zu können. Beispielsweise ist der Probenträger auch für sichtbares Licht zumindest teilweise durchlässig.

Insofern wird die vorliegende Aufgabe nach einem zweiten Aspekt der Erfindung auch von einem Probenträger für eine Vor-Ort-Analyse von Exkrementen mit einem Grundkörper gelöst, wobei der Probenkörper geometrisch unterschiedlich geformte, einzelne Probenanalysefelder aufweist.

Hierdurch können insbesondere unterschiedliche Indikatorflächen an dem Probenträger ausgestaltet werden, so dass mehrere Analysen unabhängig voneinander durchgeführt werden können.

Durch einen diesbezüglich ausgestalteten Probenträger kann die vorliegende Vorrichtung noch effizienter arbeiten.

Weiterhin offenbart wird auch ein Probenträger für eine Vor-Ort-Analyse von Exkrementen mit einem Grundkörper gelöst, wobei sowohl an der Vorderseite als auch an der Rückseite des Grundkörpers Probenanalysefelder angeordnet sind. Sind Probenanalysefelder beidseits des Grundkörpers angeordnet, kann die Anzahl von gleichzeitig durchführbaren Analysen signifikant gesteigert werden.

Durch die Probenträger können auch entsprechende Vorrichtungen zur Vor-Ort-Analyse von Exkrementen sowie diesbezügliche Verfahren zum Betreiben derartiger Vorrichtungen wesentlich weiterentwickelt werden. Beispielsweise kann durch den Einsatz entsprechender Probenträger die Anzahl an Sensoreinheiten an Vorrichtungen sinnvoll erhöht werden, so dass diesbezügliche Verfahren noch schneller durchgeführt werden können.

Bei herkömmlichen Urinteststreifen sind die Testmaterialien (Reagenz bzw. Saugpapier) stets nur von einer Seite auf Trägermaterialien (hier: Grundkörper) aufgebracht. Bei der vorliegenden Erfindung sind Probenanalysefelder von beiden Seiten auf das Trägermaterial aufgebracht. Hierdurch kann Platz gespart und der Bedarf an Trägermaterial und auch Probenträger reduziert werden.

Darüber hinaus können hinsichtlich eines einzelnen Probenträgers mehr Analysen bedenkenlos gleichzeitig durchgeführt werden, wenn einzelne Probenanalysefelder unterschiedliche Indikatoren aufweisen.

Ferner kann eine Gefahr von gegenseitiger Beeinflussung von unterschiedlichen Indikatoren reduziert werden, wenn einzelne Probenanalysefelder beanstandet voneinander an dem Grundkörper angeordnet sind.

Zum Zweck einer weiteren Optimierung kann darüber hinaus auch die Form von Probenanalysefeldern verändert werden, die bisher vornehmlich viereckig in einer Größe von 5 mm x 5 mm vorgesehen ist.

Insofern sieht eine weitere Ausführungsvariante vor, dass einzelne Probenanalysefelder kreisrund ausgestaltet sind.

Hierbei sind aber auch andere verschiedene geometrische Formen denkbar, wie etwa kleinere Viereckflächen, Kreisflächen, Ovalflächen oder dergleichen.

Sind der Grundkörper und einzelne Probenanalysefelder zumindest teilweise lichtdurchlässig, kann die Vielfalt an Analyseverfahren an einer entsprechend ausgerüsteten Vorrichtung wesentlich erhöht werden. Nur zum Beispiel sei hierbei an eine Durchlichtmessung oder dergleichen gedacht.

Ist der Probenträger von der Beschickungseinrichtung bereitgestellt und noch nicht mit Exkrement kontaminiert, ist im Sinne der Erfindung von einem neuen Probenträger die Rede.

Ist ein Probenträger jedoch mit Exkrement kontaminiert, ist vorliegend die Rede von einem benutzten bzw. gebrauchten Probenträger.

Die Begrifflichkeit "Exkrement" beschreibt im Sinne der Erfindung Körperausscheidungen, wie etwa Urin und Stuhl.

Der Begriff "Vor-Ort-Analyse" beschreibt im Sinne der Erfindung, dass eine Analyse von Exkrementen zumindest teilweise oder bevorzugt zur Gänze an dem Ort der Ausscheidung, wir beispielsweise an einer Toilette, einem Urinal oder ähnlichem erfolgen kann.

Mit anderen Worten bedeutet dies, dass das zu untersuchende Exkrement, welcher Art auch immer, für eine Analyse nicht erst aufwendig zu einem entfernten Labor eingeschickt werden muss.

Es versteht sich, dass vor Ort gewonnene Daten bzw. Informationen zur weiteren Auswertung oder zur Speicherung aber auch an externe Einrichtungen, wie etwa einem Datenträger usw., übermittelt werden können.

Jedenfalls wird zum Entnehmen einer gewünschten Probe das bewegliche Armelement der Entnahmeeinrichtung aus dem Führungsteil so weit ausgefahren, dass ein neuer, frischer Probenträger mit dem jeweiligen Exkrement in Berührung kommen kann.

Anschließend wird der mit dem Exkrement kontaminierte Probenträger zur Analyse wieder in das Führungsteil eingefahren, um dort mittels Sensoren einer Sensoreinheit der Vorrichtung untersucht werden zu können.

Eine bevorzugte Ausführungsvariante sieht vor, dass der Reinbereich des Führungsteils seitlich neben dem Kontaminationsbereich des Führungsteils angeordnet ist, wobei der Reinbereich und der Kontaminationsbereich durch eine Trennung räumlich voneinander getrennt sind, vorzugsweise durch eine starre Trennwand. Somit sind der Reinbereich und der Kontaminationsbereich, insbesondere die Führungsbahn des beweglichen Armelements, sehr deutlich voneinander getrennt, so dass die Gefahr verringert werden kann, dass eventuell an dem Armelement anhaftendes Exkrement in den Reinraum gelangt und dort einen neuen Probenträger mit altem Exkrement, ggf. einer anderen Person, verunreinigt.

Damit der Probenträger dennoch trennwandübergreifend einerseits mit seiner ersten Hälfte an dem Halter des beweglichen Armelements gehaltert werden kann (Kontaminationsbereich) und andererseits mit seiner anderen Hälfte kontaminationsfrei durch das Führungsteil (Reinbereich) der neuen Probe zugeführt werden kann, ist es zweckmäßig, wenn in dem Bereich der Trennung eine schlitzartige Öffnung angeordnet ist, durch welche hindurch ein neuer Probenträger sowohl einerseits in dem Kontaminationsbereich als auch andererseits in dem Reinbereich anordenbar ist.

Ferner ist es vorteilhaft, wenn der Reinbereich des Führungsteils in Einfahrrichtung des beweglichen Armelements gesehen axial hinter einer Löseeinheit zum Loslösen eines benutzten Probenträgers von dem beweglichen Armelement angeordnet ist. Hierdurch kann sichergestellt werden, dass der benutzte Probenträger vor einem Erreichen des Reinraums zuverlässig von dem einfahrenden Armeelement abgelöst wird, so dass bei ordnungsgemäßem Betrieb der Vorrichtung eine Kontamination des Reinraums ausgeschlossen werden kann.

Es versteht sich, dass diese schlitzartige Öffnung unterschiedlich ausgestaltet sein kann. Gegebenenfalls sind an der Trennung ausschließlich oder zusätzlich Borstenleisten vorgesehen, um eine im Wesentlichen nur für den Probenträger durchlässige Trennung zu realisieren.

Es ist möglich, die schlitzartige Öffnung derart auszubilden, dass der Probenträger im Bereich der Trennung oben und unten geführt sein kann, wodurch der Probenträger innerhalb des Führungsteils eine gewisse Höhenführung erfährt. Hierdurch können die Probenträger stets mit einem möglichst gleichen Abstand vor den Sensoren gehalten werden.

Weiterhin offenbart ist eine Vorrichtung zur Vor-Ort-Analyse von Exkrementen mit einem Gehäuse, mit einer Entnahmeeinrichtung, mittels welcher eine Probe an Exkrement entnehmbar ist, bei welcher die Entnahmeeinrichtung ein gegenüber dem Gehäuse in axialer Verschieberichtung bewegliches Armelement für einen Probenträger umfasst, welches zumindest teilweise in einem Führungsteil ein- und ausfahrbar geführt ist, und mit einer Analyseeinrichtung, mittels welcher die entnommene Probe zumindest teilweise analysierbar ist, gelöst, wobei sich die Vorrichtung durch eine Sensoreinheit mit einer Durchlichtmessapparatur zum Durchleuchten des Probenträgers auszeichnet.

Mittels einer derartigen Durchlichtmessapparatur können besonders präzise Untersuchungsergebnisse erzielt werden, insbesondere eine qualifizierte Untersuchung und Bestimmung von bzw. an Exkrementen.

Hierbei können mittels einer Durchlichtmessung insbesondere eine Messung und Auswertung eines Indikatormaterials vorteilhaft durchgeführt werden.

Insbesondere werden mittels einer entsprechend ausgestalteten Sensoreinheit bzw. der Durchlichtmessapparatur sowie einer hiermit einhergehenden Durchlichtmessung optische Weglängen für ein aus einer Lichtquelle emittiertes Licht durch eine Probe hindurch signifikant erhöht.

Vorteilhafterweise wird hierbei eine höhere Informationsdichte gewonnen, da der Probenträger bzw. ein entsprechendes Testmaterial durchleuchtet wird, und der Probenträger nicht nur oberflächlich bestrahlt wird, wie dies bisher üblich ist, etwa bei einer Reflexionsphotometrie, bei welcher mittels eines Reflexionsphotometers Eigenschaften einer Strahlung gemessen werden, welche von einer durch eine Lichtquelle angestrahlten Oberfläche reflektiert wird.

Als Testmaterial können insbesondere die bereits beschriebenen Indikatorstreifen oder - blättchen usw. herangezogen werden, aber auch eigens dafür vorgesehene Materialien genutzt werden.

Eine sehr einfache Form der Durchlichtmessung kann beispielsweise durch die Tränkung eines einfachen Aufnahmematerials etwa auf Cellulosebasis (Filterpapier) oder künstlichem Faserstoff mit Exkrementen erfolgen.

Das "Durchlicht" ermöglicht speziell die Sichtbarmachung einzelner Inhaltsstoffe in einem Aufnahmematerial für die optische Messeinheit.

Darüber hinaus können auch gängige Testreifenaufbauten genutzt werden, die beispielsweise aus Trägermaterial, Reagenzien und Saugpapier etc. bestehen.

Die Messung kann mit und ohne unterstützendem Trägermaterial erfolgen.

Als geeignete Trägermaterial für das Indikatorfeld kann üblicherweise Plastik oder auch Cellulose eingesetzt werden.

Eine Durchlichtmessung kann baulich einfach durchgeführt werden, wenn die Durchlichtmessapparatur zumindest teilweise beidseits des Probenträgers angeordnet ist.

Eine äußerst exakte Detektion kann dadurch erzielt werden, wenn die Durchlichtmessapparatur den Probenträger während der Durchlichtmessung umbaut. Das heißt, dass der Probenträger von der Sensoreinheit bzw. der Durchlichtmessapparatur umbaut ist.

Nicht nur hierzu ist es vorteilhaft, wenn die Durchlichtmessapparatur eine integrale Transportstrecke aufweist, entlang welcher der Probenträger bewegbar ist.

Die integrale Transportstrecke zeichnet sich unter anderem dadurch aus, dass sie vor unerwünschtem, kritischen Umgebungslicht geschützt ist.

Vorzugsweise ist diese Transportstrecke von der Sensoreinheit bzw. der Durchlichtmessapparatur zumindest bereichsweise umbaut, so dass auch eine sehr kompakte Bauweise insbesondere der Sensoreinheit erzielt werden kann.

Vorteilhafterweise weist die Sensoreinheit ein Gehäuse auf, in welcher die Transportstrecke der Durchlichtmessapparatur zumindest teilweise angeordnet ist, wodurch ein zu durchleuchtender Probenträger während der Durchlichtmessung etwa von Umgebungslicht oder dergleichen gut geschützt ist.

Das Gehäuse kann konstruktiv sehr einfach bereitgestellt werden, wenn es zumindest teilweise durch das Führungsteil der Entnahmeeinrichtung oder eines anderen geeigneten Bauteils der Vorrichtung ausgestaltet ist.

Darüber hinaus kann die Sensoreinheit noch kompakter gebaut werden, wenn die Durchlichtmessapparatur eine Beleuchtungseinrichtung auf einer ersten Seite der integralen Transportstrecke und eine Detektiereinrichtung auf einer der ersten Seite gegenüberliegenden, zweiten Seite der integralen Transportstrecke angeordnet ist.

Sollen etwa die Beleuchtungseinrichtung und die Detektiereinrichtung als Alternative auf einer gemeinsamen Seite der Transportstrecke angeordnet sein, könnte der Beleuchtungseinrichtung auf der anderen Seite auch eine Spiegeleinrichtung gegenüber liegen, welche Lichtstrahlen entsprechend zu der Detektiereinrichtung umlenken kann.

Die Beleuchtungseinrichtung besitzt hierbei wenigstens eine Lichtquelle, während die Detektiereinrichtung demgegenüber wenigstens eine Detektiersensorfläche aufweist.

Hierbei ist es für die hier beschriebene Durchlichtmessung vorteilhaft, wenn die wenigstens eine Lichtquelle der wenigstens einen Detektiersensorfläche gegenüberliegt.

Hinsichtlich einer anderen Ausführungsvarianten ist es aber auch möglich, dass wenigstens eine weitere Lichtquelle auf der Seite der Detektiereinrichtung angeordnet ist, um den Probenträger zusätzlich ausleuchten zu können.

Hierbei können beim Betreiben der Sensoreinheit der Detektiereinrichtung gegenüberliegende Leuchtmittel ausschließlich oder darüber hinaus kumulativ zu den weiteren Lichtquellen, welche auf Seiten der Detektiereinrichtung angeordnet sind, eingesetzt werden. Oder es werden alternativ zu den weiteren Lichtquellen, welche auf Seiten der Detektiereinrichtung angeordnet sind, ausschließlich oder kumulativ der Detektiereinrichtung gegenüberliegende Leuchtmittel eingesetzt, um gewünschte Untersuchungen durchzuführen.

Des Weiteren ist es vorteilhaft, wenn die Durchlichtmessapparatur mehr als eine Lichtquelle aufweist, wobei ein Hauptstrahlengang wenigstens einer der Lichtquellen rechtwinkelig zu einer Detektiersensorfläche und wenigstens ein weiterer Hauptstrahlengang wenigstens einer weiteren der Lichtquellen in einem hiervon abweichenden Winkel zu einer Detektiersensorfläche verlaufend angeordnet sind. Hierdurch kann die Qualität der Analyse noch weiter verbessert werden.

Es versteht sich, dass auch die übrigen hier beschriebenen Vorrichtungen und/oder Verfahren mittels einer derartige Durchlichtmessapparatur bzw. einer hierdurch ausgerüsteten Sensoreinheit vorteilhaft weiterentwickelt werden können.

Eine besonders bevorzugte Ausführungsvariante sieht vor, dass das Führungsteil eine Sensoreinheit umfasst, welche seitlich neben der Führungsbahn zum Führen des beweglichen Armeelements angeordnet ist. Hierdurch kann eine Trennung von Führung des beweglichen Armelements und Analyse einer Probe noch präziser bewerkstelligt werden.

"Seitlich" bedeutet vorliegend in Bezug auf die Schmalseite der Führungsbahn bzw. des Armelements, also rechts oder links der Führungsbahn bzw. des Armelements.

Nicht gemeint mit dem Begriff "seitlich" ist an der Oberseite oder Unterseite befindlich, also auch nicht in Überdeckung mit der Breitseite der Führungsbahn bzw. des Armelements. Und somit auch nicht oberhalb oder unterhalb der Führungsbahn bzw. des Armelements.

Ist der Reinbereich des Führungsteils in Ausfahrrichtung des beweglichen Armelements gesehen axial vor einer Sensoreinheit angeordnet, kann konstruktiv sehr einfach vermieden werden, dass beim Einfahren des benutzten Probenträgers der Reinraum durch Exkrement unbeabsichtigt kontaminiert wird, da der benutzte Probenträger lediglich nur bis zu der Sensoreinheit verlagert werden braucht.

Besonders zweckmäßig ist es, wenn der die Sensoreinheit an dem freien Ende des Führungsteils angeordnet ist, da hierdurch vermieden werden kann, dass der benutzte Probenträger weiter axial in das Führungsteil eingefahren werden muss, um das daran anhaftende Exkrement analysieren zu können.

Ist die Sensoreinheit in Einfahrrichtung des beweglichen Armelements gesehen axial vor dem Reinbereich des Führungsteils angeordnet, gelangt der benutzte Probenträger für die Analyse erst gar nicht bis in den Reinbereich des Führungsteils hinein.

Ist die Sensoreinheit in Einfahrrichtung des beweglichen Armelements gesehen axial vor einer Löseeinheit zum Lösen eines benutzten Probenträgers von dem beweglichen Armelement angeordnet ist, kann der benutzte Probenträger nach einem abgeschlossenen Analysevorgang einfach von dem Armelement gelöst werden, indem das Armelement einfach weiter in Einfahrrichtung bewegt wird.

Darüber hinaus ist es vorteilhaft, wenn die Sensoreinheit an dem freien Ende des Führungsteils eine Sensorenbereichsöffnung mit einer geringeren Öffnungshöhe als eine Führungsbahnöffnung der Führungsbahn aufweist. Aufgrund der geringeren Öffnungshöhe der Sensorenbereichsöffnung ist der Sensorenbereich gegen Verschmutzungen von außen besser geschützt. Durch die dementsprechend größeren Öffnungshöhe der Führungsbahnöffnung steht dort genügend Raumhöhe für das bewegliche Armelement und dem daran temporär gehalterten Probenträger, insbesondere für die diesbezügliche Halterung, zur Verfügung.

Kumulativ oder alternativ ist es von Vorteil, wenn die Sensorenbereichsöffnung eine Breite aufweist, welche größer ist als die Breite der Führungsbahn. Durch die breitere Sensorenbereichsöffnung kann die Hälfte des Probenträgers, welche in Überdeckung mit der Sensoreinheit gebracht werden kann, größere, insbesondere länger ausgeführt sein, als die Hälfte des Probenträgers, mittels welcher Letzterer an dem Halter des beweglichen Armelements gehaltert ist.

Eine weitere vorteilhafte Ausführungsvariante sieht vor, dass der Reinbereich des Führungsteils breiter ist als der Kontaminationsbereich des Führungsteils, insbesondere als die Führungsbahn. Hierdurch kann gewährleistet werden, dass ein neuer Probenträger an dem Führungsteil möglichst großflächig kontaminationsfrei bleibt, so dass die Gefahr einer unbeabsichtigten Verschmutzung des Probenträgers durch älteres Exkrement oder dergleichen möglichst verringert werden kann.

Zudem ist es zweckmäßig, wenn der Reinbereich des Führungsteils in einen Sensorenbereich des Führungsteils räumlich übergeht. Hierdurch lassen sich der Reinbereich und der Sensorenbereich in axialer Verschieberichtung räumlich direkt hintereinander platzieren, wodurch das Führungsteil sehr kompakt baut. Außerdem kann ein aus Richtung des Reinraums kommender neuer Probenträger über die Sensoreinheit hinweg zur Probenentnahme geführt werden, um den Probenträger vor einer Probenentnahme gegebenenfalls bereits zu registrieren, oder um zu prüfen, ob der neue Probenträger ordnungsgemäß an dem beweglichen Armelement gehaltert ist.

Damit der Reinbereich des Führungsteils die Beschickungseinrichtung zumindest teilweise überdecken und schützen kann, ist es vorteilhaft, wenn der Reinbereich des Führungsteils an seiner dem Sensorenbereich abgewandten Seite breiter als an seiner dem Sensorenbereich zugewandten Seite ausgebildet ist.

Baulich kann die Vorrichtung sehr einfach gehalten werden, wenn sowohl der Kontaminationsbereich als auch der Reinbereich in einem Haltebügel der Vorrichtung angeordnet sind. Vorteilhafterweise kann hierdurch das Führungsteil unmittelbar als Haltebügel dienen. Insbesondere kann hierdurch der Transfer eines neuen Probenträgers für eine Probenentnahme sehr einfach realisiert werden.

Vorzugsweise sind der Kontaminationsbereich und der Reinbereich in einem gemeinsamen Haltebügel angeordnet.

Sind sowohl der Kontaminationsbereich als auch der Reinbereich zumindest teilweise gekrümmt, können diese wichtigen Bereiche ohne Weiteres in einen Haltebügel der Vorrichtung integriert werden.

Eine weitere auch ohne die übrigen Merkmale der Erfindung vorteilhafte Ausführungsvariante sieht eine Sensoreinheit mit einem Thermosensor zum Detektieren einer Exkrementtemperatur, einer Körpertemperatur, eines Exkrementvolumens und/oder eines Exkrementvolumenstroms vor.

Vorteilhafterweise lassen sich durch an der Vorrichtung angebrachte Temperatursensoren, wie beispielsweise Infrarotsensoren oder dergleichen, relevante Teile eines Toiletteninnenraums scannen, und so weitere Informationen gewinnen.

Dadurch, dass entsprechende Thermosensoren bevorzugt an der vorliegenden Vorrichtung angeordnet sind, brauchen sie einerseits nicht fest an der Toilette verbaut zu sein. Und andererseits sind die Thermosensoren mobil an der Toilette angebracht, so dass sie an bestehenden Toiletten vorteilhaft nachgerüstet werden können.

So kann eine direkte Temperaturmessung des Exkrements direkt bei Verlassen des Körpers oder nach Kontakt mit der Toilette Rückschlüsse auf die Körpertemperatur der Person zulassen.

Durch geeignete Rechenmodelle und/oder Algorithmen können hierbei etwaige Temperaturverluste bedingt durch Außenluft oder Toilettenkontakt zurückgerechnet bzw. kompensiert werden.

Hierdurch lassen sich Körpertemperaturschwankungen erkennen und es lassen sich beispielsweise Rückschlüsse auf Krankheiten ziehen.

Eine Messung des Temperaturverlaufs während der gesamten Exkretionsphase ermöglicht des Weiteren eine Rückrechnung des Volumenstroms und damit auch der abgegebenen Menge.

Darüber hinaus erschließt sich auch die Charakteristik des Entleerens, was bei manchen Krankheitsbildern ebenfalls eine interessante Information ist. Beispielsweise nach Nierentransplantationen oder bei Harnsteinen.

Vorteilhafterweise kann mit denselben Thermosensoren kumulativ auch eine Messauslösung über die Feststellung einer Temperaturveränderung innerhalb der Toilette bedingt durch zugeführte Exkremente erreicht werden.

Weiterhin offenbart ist eine Vorrichtung zur Vor-Ort-Analyse von Exkrementen mit einem Gehäuse, mit einer Entnahmeeinrichtung, mittels welcher eine Probe an Exkrement entnehmbar ist, bei welcher die Entnahmeeinrichtung ein gegenüber dem Gehäuse bewegliches Armelement für einen Probenträger umfasst, welches zumindest teilweise in einem Führungsteil ein- und ausfahrbar geführt ist, mit einer Beschickungseinrichtung, mittels welcher ein neuer Probenträger für die Entnahmeeinrichtung bereitstellbar ist, und mit einer Analyseeinrichtung, mittels welcher die entnommene Probe zumindest teilweise analysierbar ist, gelöst, wobei sich die Vorrichtung dadurch auszeichnet, dass die Entnahmeeinrichtung eine Löseeinheit mit Abstreifmitteln zum Lösen eines benutzten Probenträgers von dem Armelement umfasst.

Mittels derartiger Abstreifmittel kann ein benutzter Probenträger sehr zuverlässig von dem Halter des beweglichen Armelements entfernt werden, so dass die Vorrichtung zuverlässiger betrieben werden kann.

Insbesondere kann hierbei die Gefahr zur Gänze ausgeschlossen oder zumindest signifikant verringert werden, dass Exkremente allgemein, insbesondere Exkremente eines anderen Benutzers, unbeabsichtigt tiefer in das Führungsteil gelangen kann, beispielsweise dadurch, dass ein benutzter Probenträger nicht richtig aus dem Halter des beweglichen Armelements entfernt ist. Jedenfalls sinkt durch die vorliegenden Abstreifmittel die Gefahr, dass Analyseergebnisse verfälscht werden.

Insofern ist ein ständiges mobiles Monitoring mittels Urin- und/oder Stuhlproben noch einfacher und zuverlässiger durchführbar.

Um die Zuverlässigkeit der vorliegenden Löseeinheit zu erhöhen, ist es vorteilhaft, wenn die Löseeinheit an dem Führungsteil, insbesondere in einem Übergangsbereich auf axialer Höhe zwischen einem Sensorenbereich des Führungsteils und einem Reinbereich des Führungsteils angeordnet ist. Hierdurch kann erreicht werden, dass ein benutzter Probenträger mittels des beweglichen Armelements noch bis in den Sensorenbereich hinein verlagert werden kann, aber weiter in Richtung auf den Reinbereich zu zuverlässig von dem Halter des beweglichen Armelements gelöst wird, bevor der benutzte Probenträger weiter in den Reinraum eindringen kann.

Eine besonders bevorzugte Ausführungsvariante sieht vor, dass die Löseeinheit derart ausgestaltet ist, dass die Löseeinheit von einem an dem beweglichen Armelement angeordneten Halter zum temporären Haltern eines Probenträgers und/oder Aktivierungsmitteln zum Aktivieren der Beschickungseinrichtung passierbar ist. Hierdurch kann gewährleistet werden, dass ein benutzter Probenträger zuverlässig von dem beweglichen Armelement gelöst wird, aber das Armelement trotz daran angeordneten Halter und Aktivierungsmittel dennoch weiter in Einfahrrichtung in das Führungsteil eingefahren werden kann, um einen neuen Probenträger betriebssicher übernehmen zu können.

In diesem Zusammenhang ist es auch vorteilhaft, wenn die Löseeinheit einen von dem beweglichen Armelement durchdringbaren Grundkörper aufweist, wobei der Grundkörper zumindest teilweise einen Raum mit einer Raumhöhe umgrenzt, welche um ein Vielfaches größer ist, als die Armelementdicke. Durch eine derartige Raumhöhe können insbesondere der Halter für die Probenträger und die vorstehend bereits erwähnten Aktivierungsmittel oder dergleichen die Löseeinheit problemlos passieren.

Es versteht sich, dass die Raumhöhe insbesondere in Abhängigkeit von der Ausgestaltung des Halters bzw. der Aktivierungsmittel und auch im Allgemeinen von der Gestalt des beweglichen Armelements gewählt werden kann.

Es hat sich bereits bewährt, wenn die Raumhöhe des Grundkörpers mehr als das Doppelte, vorzugsweise mehr als das Zehnfache, der Armelementdicke beträgt. Insbesondere eine Raumhöhe von mehr als das Zehnfache der Armelementdicke, wie etwas das Fünfzehnfache, hat sich als hinreichend hoch erwiesen, so dass ein Zusammenspiel zwischen Abstreifmittel und Probenträger als auch ein ungehindertes Passieren von Halter und Aktivierungsmittel gewährleistet ist.

Es versteht sich, dass die im Sinne der Erfindung geeigneten Abstreifmittel vielfältig ausgestaltet sein können.

Beispielsweise können die Abstreifmittel Bürsten und/oder Borsten umfassen, mittels welchen ein Probenträger aus dem Halter des beweglichen Armelements abgestreift werden.

In Praxistests hat sich bewährt, wenn die Abstreifmittel mindestens ein flexibles Fingerelement umfassen, an welchem das bewegliche Armelement, bevorzugt kontaktbehaftet, translatorisch vorbeiführt ist. Hierbei kann das mindestens eine flexible Fingerelement den benutzten Probenträger zurückhalten, wenn das bewegliche Armelement in axialer Einfahrrichtung weiter in das Führungsteil eingefahren wird, so dass der benutzte Probenträger letztendlich aus dem Halter gelöst wird und anschließend beispielsweise in die Toilettenschüssel fällt und darüber entsorgt wird.

Hierbei kann das mindestens eine flexible Fingerelement in Wirkkontakt mit dem beweglichen Armelement stehen. Oder es ist um einen geringen Abstand oberhalb des beweglichen Armelements positioniert.

Das mindestens eine Fingerelement erstreckt sich hierbei ausgehend von dem Grundkörper der Löseeinheit in Richtung der vorstehend bereits beschriebenen Führungsbahnöffnung des Führungsteils und knickt dabei in Richtung auf das bewegliche Armelement ab, so dass das freie Ende des Fingerelements auf das bewegliche Armelement zeigt, entweder mit einer Berührung zwischen dem freien Ende des Fingerelements und dem Armelement oder berührungsfrei.

So kann ein von der Beschickungseinrichtung kommender neuer Probenträger die Löseeinheit problemlos passieren, wenn das bewegliche Armelement in Ausfahrrichtung bewegt wird. Wird das bewegliche Armelement jedoch wieder eingezogen und hierbei in Einfahrrichtung bewegt, bleibt der zwischenzeitlich benutzte Probenträger an dem freien Ende des Fingerelements hängen und wird durch Weiterbewegung des beweglichen Armelements aus dem Halter gelöst.

Insofern besitzt die Löseeinheit eine Loslösespitze, die in Richtung auf Führungsbahnöffnung des Führungsteils gerichtet ist.

Eine weitere sehr vorteilhafte Ausführungsvariante sieht vor, dass das flexible Fingerelement zweigeteilt ist, und zwei durch eine Lücke voneinander beabstandete flexible Fingerteile aufweist. Hierdurch kann der Probenträger über die Breite des beweglichen Armelements gesehen gut von dem flexiblen Finderelement erfasst und gelöst werden, wobei das flexible Fingerelement an sich dennoch sehr filigran ausgebildet sein kann.

Sind die zwei flexiblen Fingerteile quer zur axialen Verschieberichtung bzw. translatorischen Verlagerungsrichtung des beweglichen Armelements voneinander beabstandet angeordnet, können gegebenenfalls höher bauende Spezifikationen des beweglichen Armelements zwischen diesen zwei flexiblen Fingerteilen hindurch die Löseeinheit passieren.

Bevorzugt sind die Abstreifmittel an wenigstens zwei Seiten des beweglichen Armelements angeordnet, so dass ein benutzter Probenträger auch von zwei Seiten penetriert werden kann, wodurch die Sicherheit des Lösens des Probenträgers aus dem Halter des beweglichen Armelements noch weiter erhöht werden kann.

Bevorzugt sind die Abstreifmittel an der Oberseite und der Unterseite des Armelements angeordnet, so dass der benutzte Probenträger betriebssicher erfasst und gelöst von dem Halter bzw. und somit von dem Armelement abgestreift werden kann.

Sind nun jeweils ein flexibles Fingerelement mit jeweils zwei flexiblen Fingerteilen an der Oberseite und an der Unterseite des beweglichen Armelements platziert, kann ein benutzter Probenträger mit vier Abstreifmitteln erfasst und aus dem Halter gelöst werden. Bei dieser zuletzt genannten Ausgestaltung der Löseeinheit bilden insgesamt mindestens vier flexible Fingerteile eine Loslösespitze der Löseeinheit, die in Richtung auf Führungsbahnöffnung des Führungsteils gerichtet ist.

Weiterhin offenbart ist eine Vorrichtung zur Vor-Ort-Analyse von Exkrementen mit einem Gehäuse, mit einer Entnahmeeinrichtung, mittels welcher eine Probe an Exkrement entnehmbar ist, bei welcher die Entnahmeeinrichtung ein gegenüber dem Gehäuse bewegliches Armelement für einen Probenträger umfasst, welches zumindest teilweise in einem Führungsteil ein- und ausfahrbar geführt ist, mit einer Beschickungseinrichtung, mittels welcher ein neuer Probenträger für die Entnahmeeinrichtung bereitstellbar ist, und mit einer Analyseeinrichtung, mittels welcher die entnommene Probe zumindest teilweise analysierbar ist, gelöst, wobei sich die Vorrichtung dadurch auszeichnet, dass die Beschickungseinrichtung mittels Bewegung des beweglichen Armelements aktivierbar ist.

Dadurch, dass die Beschickungseinrichtung mit Hilfe der Bewegung des beweglichen Armelements aktiviert, betrieben und gesteuert werden kann, kann die Konstruktion der vorliegenden Vorrichtung nochmals vereinfacht und dadurch auch zuverlässiger betrieben werden.

Insofern zeichnet sich die vorliegende Vorrichtung dadurch aus, dass für das Betreiben der Beschickungseinrichtung die Antriebseinheit verwendet wird, mittels welcher das bewegliche Armelement angetrieben ist.

Insofern besitzen die Entnahmeeinrichtung und die Beschickungseinrichtung den selben Antrieb.

Mit anderen Worten zeichnet sich die vorliegende Erfindung auch unabhängig von den übrigen Merkmalen der Erfindung dadurch aus, dass die Entnahmeeinrichtung und die Beschickungseinrichtung eine einzige Antriebseinheit aufweisen.

Darüber hinaus kann die Verwendung der vorliegenden Vorrichtung noch weiter verbessert werden, wenn die Vorrichtung ein automatisches Miktionstagebuch aufweist, mittels welchem Messdaten speicherbar sind.

Vorteilhafterweise lassen sich ermittelte Informationen, wie beispielsweise Urinparameter, Uhrzeit der Exkrementabgabe, Dauer der Exkrementabgabe oder ähnliches, in einem digitalen Miktionstagebuch unmittelbar festhalten. Die Informationen können insbesondere zur Weiterverarbeitung an digitale Endgeräte oder medizinische Systeme übermittelt werden.

Speziell in dem Zusammenhang mit den Merkmalen des vorliegenden Halters wird die Aufgabe der Erfindung auch noch von einem Verfahren zum Betreiben einer Vorrichtung zur Vor-Ort-Analyse von Exkrementen gelöst, bei welchem ein Probenträger mittels eines axial beweglichen Armelements für eine Probenentnahme gegenüber einer Sensoreinheit linear verfahren wird, bei welchem der Probenträger an einem Halter des axial beweglichen Armelements gehaltert wird und bei welchem eine von dem Probenträger getragene Probe an Exkrement an der Vorrichtung zumindest teilweise analysiert wird, wobei sich das Verfahren dadurch auszeichnet, dass dem Halter ein neuer Probenträger quer zur axialen Verschieberichtung des axial beweglichen Armelements zugeführt wird, indem eine Beschickungseinrichtung mittels der in axialer Verschieberichtung erfolgten Axialbewegung des axial beweglichen Armelements angetrieben wird.

Mittels des hier vorgeschlagenen Verfahrens kann die Vorrichtung besonders effizient betrieben werden, da auf für mehrere Funktionen auf ein und denselben Antrieb zugegriffen werden kann.

Konstruktiv kann hierbei das Zusammenwirken von beweglichen Armelement und der Beschickungseinrichtung auf verschiedene Art gelöst werden.

Eine baulich besonders einfache Ausführungsvariante sieht vor, dass die Beschickungseinrichtung einen mittels einer Bewegung des beweglichen Armelements aktivierbaren Aktivierungshebel aufweist. Dieser Aktivierungshebel kann dann durch die Bewegung des beweglichen Armelements betätigt werden, bevorzugt während sich das bewegliche Armelement in axialer Einfahrrichtung bewegt.

Speziell in diesem Zusammenhang wird die Aufgabe der Erfindung auch noch von einem Verfahren zum Betreiben einer Vorrichtung zur Vor-Ort-Analyse von Exkrementen gemäss Anspruch 10 gelöst.

Mittels des hier vorgeschlagenen Verfahrens kann die Vorrichtung besonders effizient betrieben werden, da auf einen zusätzlichen Antrieb zum Lösen eines benutzen Probenträgers aus dem Halter verzichtet werden kann.

Ein auf Basis des durch das bewegliche Armelement aktivierbaren Aktivierungshebels arbeitender Aktivierungs- bzw. Betätigungsmechanismus für die Beschickungseinrichtung kann konstruktiv einfach gehalten werden, wenn der Aktivierungshebel um eine Drehachse drehbar gelagert ist, wobei die Drehachse für den Aktivierungshebel mittels einer Bewegung des Armelements verschieblich ist. Hierdurch kann die Beschickungseinrichtung translatorisch bewegt werden, während der Aktivierungshebel um die Drehachse dreht.

Insofern ist es vorteilhaft, wenn die Drehachse für den Aktivierungshebel quer zu der axialen Bewegungsrichtung des Armelements verschieblich ist.

Der Aufbau der Beschickungseinrichtung kann vorliegend sehr kompakt realisiert werden, wenn die Beschickungseinrichtung eine Bewegungskurve aufweist, entlang welcher der Aktivierungshebel abrollbar ist, sobald der Aktivierungshebel um eine Drehachse des Aktivierungshebels dreht. Hierdurch kann der Aktivierungshebel einerseits an der Drehachse und andererseits zusätzlich mit Hilfe der Bewegungskurve geführt sein.

Weist die Beschickungseinrichtung eine Bewegungskurve auf, entlang welcher der Aktivierungshebel abrollbar ist, wenn eine Drehachse des Aktivierungshebels quer zu der axialen Bewegungsrichtung des Armelements verschoben wird, kann der Aktivierungshebel in Richtung des beweglichen Armelements geschleppt werden, während der Aktivierungshebel von dem beweglichen Armelement um die Drehachse gedreht wird.

An dieser Stelle sei erwähnt, dass eine derartige Bewegungskurve durch unterschiedlichen Mittel zum Aufzwingen einer Bewegung bzw. einer Bewegungsbahn realisiert sein kann, wie etwa eine Kulissenführung oder dergleichen. Des Weiteren ist es vorteilhaft, wenn die Beschickungseinrichtung einen translatorisch verschieblichen Transportschlitten aufweist, mittels welchem ein neuer Probenträger einem an dem beweglichen Armelement angeordneten Halter in Zuführrichtung zuführbar ist. Durch die translatorische Verschieblichkeit des Transportschlittens kann Aufbau der vorliegenden Beschickungseinrichtung sehr vereinfacht werden.

Ist der Transportschlitten mittels einer Bewegung des beweglichen Armelements verlagerbar, kann auf einen zusätzlichen Antrieb für den Transportschlitten verzichtet werden.

Der Transportschlitten kann mittels des Aktivierungshebels quer zu der axialen Verschieberichtung des beweglichen Armelements geschleppt werden, wenn der Transportschlitten eine Drehachse für den Aktivierungshebel aufweist.

Insofern ist es vorteilhaft, wenn der Transportschlitten mittels des Aktivierungshebels quer zu der axialen Verschieberichtung des beweglichen Armelements und somit in Zuführrichtung auf einen Halter zum Haltern eines neuen Probenträgers zu verlagerbar ist. Hierdurch kann der konstruktive Aufwand an der Beschickungseinrichtung erfreulicherweise sehr gering gehalten werden.

Es ist aber nicht nur vorteilhaft, wenn der Transportschlitten translatorisch auf das bewegliche Armelement zu bewegt werden kann, sondern darüber hinaus auch, wenn der Transportschlitten entlang einer Kurvenbahn führbar ist, um eine Schneidbewegung zum Konfektionieren eines neuen Probenträgers senkrecht zu der Zuführrichtung auszuführen. Hierdurch können sogleich Schneidmittel mittels der Antriebseinheit der Entnahmeeinrichtung bzw. insbesondere des beweglichen Armelements angetrieben werden, was auch ohne die übrigen Merkmale der vorliegenden Erfindung äußerst vorteilhaft ist.

Es versteht sich, dass auch diese Kurvenbahn durch verschiedene Mittel zum Aufzwingen einer Bewegung bzw. einer Bewegungsbahn mechanisch realisiert sein kann. Auch hierbei sei an Kulissenführungen oder dergleichen gedacht.

In diesem Zusammenhang ist es vorteilhaft, wenn der Transportschlitten Schneidmittel zum Abschneiden eines neuen Probenträgers von einem Probenträgerband umfasst. Somit kann auf eine zusätzliche Schneideinrichtung verzichtet werden.

Vielmehr können die Schneidmittel bereits durch den Transportschlitten ausgestaltet sein, oder aber der Transportschlitten verfügt über entsprechende Aufnahmen, an welchen Schneidmittel vorzugsweise auswechselbar anordenbar sind.

Insofern wird die Aufgabe der Erfindung auch noch von einem Verfahren zum Betreiben einer Vorrichtung zur Vor-Ort-Analyse von Exkrementen gelöst, bei welchem ein Probenträger mittels eines axial beweglichen Armelements für eine Probenentnahme gegenüber einer Sensoreinheit linear verfahren wird, bei welchem der Probenträger an einem Halter des axial beweglichen Armelements gehaltert wird und bei welchem eine von dem Probenträger getragene Probe an Exkrement an der Vorrichtung zumindest teilweise analysiert wird, wobei sich das Verfahren dadurch auszeichnet, dass ein neuer Probenträger von einem Rollenmaterial abgetrennt wird, indem eine Schneideinrichtung mittels der in axialer Verschieberichtung erfolgten Axialbewegung des Armelements angetrieben wird.

Auch durch dieses Verfahren kann die Vorrichtung äußerst effizient betrieben werden, da auf einen zusätzlichen Antrieb für eine Schneideinrichtung verzichtet werden kann.

Als Rollenmaterial sei beispielsweise an ein gegebenenfalls mit Chemikalien getränktes Filterpapier oder dergleichen gedacht.

Das Rollenmaterial kann etwa ca. 35 cm lang und 0,5 cm breit sein.

Darüber hinaus sieht eine weitere bevorzugte Ausführungsvariante vor, dass das bewegliche Armelement Aktivierungsmittel zum Aktivieren der Beschickungseinrichtung umfasst. Durch die Aktivierungsmittel an dem Armelement kann die Beschickungseinrichtung konstruktiv einfach durch einfache Bewegung des Armelements aktiviert werden. Insofern kann der Antrieb des beweglichen Armelements genutzt werden, die Beschickungseinrichtung zu betreiben und steuern.

Im Sinne der Erfindung können diese Aktivierungsmittel unterschiedlich verwirklicht sein.

Eine sehr einfache Umsetzung der Aktivierungsmittel kann darin gesehen werden, wenn die Aktivierungsmittel eine Erhebung an der Oberfläche des beweglichen Armelements umfassen.

Eine derartige Erhebung kann unmittelbar durch das bewegliche Armelement verkörpert sein, wodurch die vorliegende Konstruktion weiter vereinfacht werden kann.

Kumulativ oder alternativ kann die Erhebung durch ein weiteres Bauteil realisiert sein, welches in geeigneter Weise an das bewegliche Armelement angebracht ist, beispielsweise durch eine Schraub- und/oder Klebeverbindung oder dergleichen.

Hierbei kann die Erhebung etwa durch ein zusätzliches Stiftteil oder ähnlichem an dem beweglichen Armelement bereitgestellt werden.

Eine weitere besonders bevorzugte Ausführungsvariante sieht vor, dass das bewegliche Armelement ein durch eine Bewegung des beweglichen Armelements betätigbaren Halter umfasst, wobei ein derartiger Halter auch ohne die übrigen Merkmale der Erfindung vorteilhaft ist, da er bisher bekannte gattungsgemäße Vorrichtungen allein für sich gesehen bereits vorteilhaft weiterentwickelt.

Jedenfalls wird zum Betätigen des Halters die Bewegung des beweglichen Armelements genutzt, so dass ein zusätzlicher Antrieb für den Halter entfallen kann.

Der vorliegende Halter basiert insofern im Wesentlichen auf einen Festklemmmmechanismus, mittels welche ein Probenträger reversibel an dem beweglichen Armelement gehaltert bzw. geklemmt werden kann.

Der Halter kann hierbei auf das bewegliche Armelement angebracht, insbesondere aufgesteckt oder festgeklippt, sein, oder der Halter ist bevorzugt direkt mittels des beweglichen Armelements verkörpert.

Als Material für den Halter kommt beispielsweise Kunststoff, Metall oder gegebenenfalls auch ein anderer Werkstoff, oder eine Kombination hieraus in Betracht.

Jedenfalls ist es zweckmäßig, wenn der Halter wenigstens zwei Klemmelemente umfasst, von denen zumindest ein Klemmelement aktiv betätigbar ist.

Des Weiteren ist es vorteilhaft, wenn die Vorrichtung eine Halterbetätigung zum Betätigen eines an dem beweglichen Armelement angeordneten Halters zum temporären Haltern des Probenträgers aufweist, wobei die Halterbetätigung ein Rampenelement umfasst. Hierbei kann der Halter mittels der Bewegung des beweglichen Armelements über dieses Rampenelement gezogen werden, wodurch zumindest ein Klemmelement elastisch ausgelenkt wird, um einen neuen Probenträger zwischen den wenigstens zwei Klemmelementen klemmen und somit an dem beweglichen Armelement haltern zu können. Speziell in dem Zusammenhang mit den Merkmalen des vorliegenden Halters wird die Aufgabe der Erfindung auch noch von einem Verfahren zum Betreiben einer Vorrichtung zur Vor-Ort-Analyse von Exkrementen gelöst, bei welchem ein Probenträger mittels eines axial beweglichen Armelements für eine Probenentnahme gegenüber einer Sensoreinheit linear verfahren wird, bei welchem der Probenträger an einem Halter des axial beweglichen Armelements gehaltert wird und bei welchem eine von dem Probenträger getragene Probe an Exkrement an der Vorrichtung zumindest teilweise analysiert wird, wobei sich das Verfahren dadurch auszeichnet, dass der Halter mittels der in axialer Verschieberichtung erfolgten Axialbewegung des axial beweglichen Armelements geöffnet oder geschlossen wird.

Mittels des hier vorgeschlagenen Verfahrens kann die Vorrichtung besonders effizient betrieben werden, da auf einen zusätzlichen Antrieb zum Betätigen des Halters verzichtet werden kann.

Weiterhin offenbart ist ein Verfahren zum Betreiben einer Vorrichtung zur Vor-Ort-Analyse von Exkrementen, bei welchem ein Probenträger mittels eines axial beweglichen Armelements für eine Probenentnahme gegenüber einer Sensoreinheit linear verfahren wird, bei welchem der Probenträger an einem Halter des axial beweglichen Armelements gehaltert wird und bei welchem eine von dem Probenträger getragene Probe an Exkrement an der Vorrichtung zumindest teilweise analysiert wird, wobei sich das Verfahren dadurch auszeichnet, dass ein mit Exkrement versehener Probenträger zwischen einer Beleuchtungseinrichtung und einer Detektiereinrichtung verbracht und für eine Durchlichtmessung mittels der Beleuchtungseinrichtung durchleuchtet wird.

Eine besonders hohe Analysequalität kann hierbei erzielt werden, wenn der mit Exkrement versehene Probenträger während der Durchlichtmessung ruht.

Hingegen kann das Verfahren zügiger durchgeführt werden, wenn der mit Exkrement versehene Probenträger während der Durchlichtmessung bewegt wird.

Hinsichtlich zusätzlicher vorteilhafter Verfahrensschritte wird auf die Beschreibung insbesondere der Vorrichtung mit der Durchlichtmessapparatur verwiesen, um vorliegend Wiederholungen zu vermeiden.

Eine weitere Verfahrensvariante sieht vor, dass eine Benetzung des Probenträgers mit Exkrement, insbesondere mit Urin, mittels zusätzlicher Bewegungen, insbesondere in axialer Aus- und/oder Einfahrrichtung, des axial beweglichen Armelements unterstützt wird. Hierdurch kann die Zeitdauer einer Probenentnahme erheblich verkürzt werden.

So ist es hinsichtlich einer anderen Verfahrensvariante auch vorteilhaft, wenn die Dauer einer Probenentnahme zwischen 10 s und 1 s, vorzugsweise zwischen 2 s bis 3 s, beträgt. Durch eine derart kurze Zeitspanne für die Probenentnahme kann die Benutzung der Vorrichtung zeitlich sehr stark begrenzt werden. Außerdem sinkt hierdurch die Gefahr einer unerwünschten Kontamination der Probe durch äußere Einflüsse, denn je schneller die entnommene Probe der Sensoreinheit innerhalb des Führungsteils zugeführt werden kann, umso geringer ist die Gefahr einer unerwünschten äußeren Einflussnahme.

Darüber hinaus ist es vorteilhaft, wenn überschüssiges Exkrement, insbesondere überschüssiger Urin, durch Vibration des axial beweglichen Armelements von diesem beweglichen Armelement, dem Probenträger und/oder dem Halter entfernt wird. Durch ein Entfernen von überschüssigem Exkrement kann die Kontaminationsgefahr der Vorrichtung, insbesondere des Führungsteils, zusätzlich gut verringert werden, da idealerweise nur eine geringe Menge an Exkrement für die Sensoruntersuchung an dem Probenträger anhaftet.

Hinsichtlich der vorliegend beschriebenen Verfahren sei an dieser Stelle noch erwähnt, dass die einzelnen Verfahren zusätzlich noch durch Vorrichtungsmerkmale ergänzt werden können, um Verfahrensabläufe weiter zu spezifizieren.

Die vorliegende Vorrichtung kann baulich noch kompakter ausgeführt werden, wenn das Führungsteil eine Gelenkverbindung aufweist, mittels welcher das Führungsteil gelenkig an dem Gehäuse angeordnet ist. Mittels dieser Gelenkverbindung kann das Führungsteil bei Nichtgebrauch an das Gehäuse angeklappt werden.

Weiterhin offenbart ist ein Haltebügel für eine Vorrichtung zur Vor-Ort-Analyse von Exkrementen zum Anbringen an einer Toilette, einem Urinal oder dergleichen, mit einer Führungsbahn, entlang welcher ein bewegliches Armelement des Haltebügels ein- und ausfahrbar geführt ist, gelöst, wobei der Haltebügel einen Kontaminationsbereich mit der Führungsbahn zum Führen des beweglichen Armeelements und darüber hinaus einen Reinbereich aufweist, in welchem ein neuer Probenträger zumindest teilweise neben der Führungsbahn anordenbar ist.

Durch einen derart strukturierten Haltebügel ist die Gefahr einer unbeabsichtigten Kontamination eines neuen Probenträgers durch alte Exkremente signifikant reduziert.

Die hier vorgeschlagenen Vereinfachungen der Vorrichtung bewirken nicht nur eine höhere Zuverlässigkeit der Vorrichtung, sondern reduzieren durch die hiermit einhergehende Bauteilreduzierung auch eine Gewichtsersparnis, sowie einen äußerst effizienteren Betrieb der Vorrichtung.

Insbesondere kann hierbei die Gefahr zur Gänze ausgeschlossen oder zumindest signifikant verringert werden, dass ein neuer Probenträger nicht oder nicht vollständig in einen Halter zum Halten des Probenträgers eingelegt wird.

Insofern ist ein ständiges mobiles Monitoring mittels Urin- und/oder Stuhlproben noch einfacher und zuverlässiger durchführbar.

Es versteht sich, dass die Merkmale der vorstehend bzw. in den Ansprüchen beschriebenen Lösungen gegebenenfalls auch kombiniert werden können, um die vorliegend erzielbaren Vorteile und Effekte entsprechend kumuliert umsetzen zu können

An dieser Stelle sei noch erwähnt, dass im Rahmen der hier vorliegenden Patentanmeldung der Ausdruck "insbesondere" immer so zu verstehen sei, dass mit diesem Ausdruck ein optionales, bevorzugtes Merkmal eingeleitet wird. Der Ausdruck ist nicht als "und zwar" und nicht als "nämlich" zu verstehen.

Ferner sei darauf hingewiesen, dass im Rahmen der hier vorliegenden Patentanmeldung unbestimmte Artikel und unbestimmte Zahlenangaben wie "ein...", "zwei..." usw. im Regelfall als mindestens-Angaben zu verstehen sein sollen, also als "mindestens ein...", "mindestens zwei..." usw., sofern sich nicht etwa aus dem Kontext oder dem konkreten Text einer bestimmten Stelle ergibt, dass etwa dort nur "genau ein...", "genau zwei..." usw. gemeint sein soll.

Zusätzlich sind weitere Merkmale, Effekte und Vorteile vorliegender Erfindung anhand anliegender Zeichnung und nachfolgender Beschreibung erläutert, in welchen beispielhaft eine Vorrichtung zur Vor-Ort-Analyse von Exkrementen dargestellt und beschrieben ist.

Komponenten, welche in den einzelnen Figuren wenigstens im Wesentlichen hinsichtlich ihrer Funktion übereinstimmen, können hierbei mit gleichen Bezugszeichen gekennzeichnet sein, wobei die Komponenten nicht in allen Figuren beziffert und erläutert sein müssen.

Es sei hier gesagt, dass es sich bei den gezeigten Figuren um Darstellungen handelt, welche den prinzipiellen Aufbau und die prinzipielle Funktionsweise illustrieren.

In der Zeichnung zeigen:
- Figur 1: schematisch eine erste perspektivische Ansicht einer teilweise dargestellten Vorrichtung zur Vor-Ort-Analyse von Exkrementen in einer Startposition;
- Figur 2: schematisch eine zweite perspektivische Ansicht der in der Figur 1 dargestellten Vorrichtung mit einem Magazin umfassend eine Materialrolle für neue Probenträger;
- Figur 3: schematisch eine dritte perspektivische Ansicht der in den Figuren 1 und 2 dargestellten Vorrichtung in einer Beschickungsposition, bei welcher dem Halter des beweglichen Armeelements ein Probenträger zugeführt wird;
- Figur 4: schematisch eine vierte perspektivische Ansicht der in den Figuren 1 bis 3 dargestellten Vorrichtung in einer Schneidposition, bei welcher der dem Halter teilweise zugeführte Probenträger von der Materialrolle abgetrennt wird;
- Figur 5: schematisch eine weitere Ansicht der in den Figuren 1 bis 4 dargestellten Vorrichtung mit Gehäuse und daran angeklapptem Führungsteil;
- Figur 6: schematisch eine Ansicht der Entnahmeeinrichtung der in den Figuren 1 bis 5 dargestellten Vorrichtung mit dem teilweise aufgewickelten beweglichen Armelement und dessen Antrieb;
- Figur 7: schematisch eine weitere perspektivische Ansicht der in den Figuren 1 bis 6 dargestellten Vorrichtung mit angeklapptem Führungsteil;
- Figur 8: schematisch eine Unteransicht des Führungsteils der in den Figuren 1 bis 7 dargestellten Vorrichtung mit Blick auf den Kontaminationsbereich, den Reinbereich sowie dem Sensorenbereich des Führungsteils;
- Figur 9: schematisch eine erste perspektivische Detailansicht der Löseeinheit der in den Figuren 1 bis 8 dargestellten Vorrichtung;
- Figur 10: schematisch eine zweite perspektivische Detailansicht der Löseeinheit der in den Figuren 1 bis 9 dargestellten Vorrichtung;
- Figur 11: schematisch eine dritte perspektivische Detailansicht der Löseeinheit der in den Figuren 1 bis 10 dargestellten Vorrichtung;
- Figur 12: schematisch eine vierte perspektivische Detailansicht der Löseeinheit der in den Figuren 1 bis 11 dargestellten Vorrichtung;
- Figur 13: schematisch eine Unteransicht eines alternativen Halters zum temporären Haltern eines Probenträgers an einem beweglichen Armelement;
- Figur 14: schematisch eine Aufsicht einer Anordnung bestehend aus der in den Figuren 1 bis 12 dargestellten Vorrichtung und einer Toilette;
- Figur 15: schematisch eine Detailansicht der in der Figur 14 gezeigten Anordnung;
- Figur 16: schematisch eine Ansicht einer vorteilhaften Sensoreinheit mit einer Durchlichtmessapparatur;
- Figur 17: schematisch eine weitere Ansicht der Sensoreinheit aus der Figur 16;
- Figur 18: schematisch eine Ansicht eine alternative Sensoreinheit mit einer Durchlichtmessapparatur;
- Figur 19: schematisch eine Aufsicht eines Probenträgers mit vereinzelten runden und ovalen Probenanalysefeldern;
- Figur 20: schematisch eine Seitenansicht eines Probenträgers mit beidseits angeordneten, vereinzelten Probenanalysefeldern; und
- Figur 21: schematisch eine Seitenansicht eines weiteren Probenträgers mit beidseits angeordneten, unterschiedlich großen, vereinzelten Probenanalysefeldern.

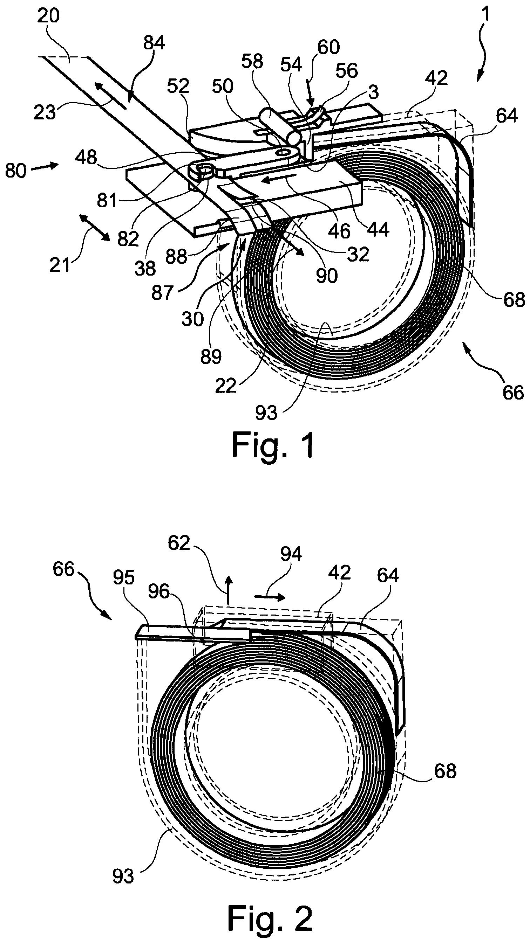

Die hinsichtlich ihres Aufbaus und Funktionsweise prinzipiell in den Figuren 1 bis 15 gezeigte Vorrichtung 1 zur Vorortanalyse von Exkrementen besteht im Wesentlichen aus einer Entnahmeeinrichtung 2, mittels welcher eine Probe an Exkrement durch einen Probenträgers 3 entnehmbar ist, aus einer Beschickungseinrichtung 4, mittels welcher ein neuer Probenträger 3 für die Entnahmeeinrichtung 2 bereitstellbar ist, sowie aus einer Analyseeinrichtung 5, mittels welcher eine entnommene Probe zumindest teilweise direkt mit der Vorrichtung 1 analysierbar ist.

Die Vorrichtung 1 weist ein Gehäuse 6 auf, an welchem mittels einer Drehgelenkverbindung 7 ein Haltebügel 10 an- bzw. abklappbar befestigt ist.

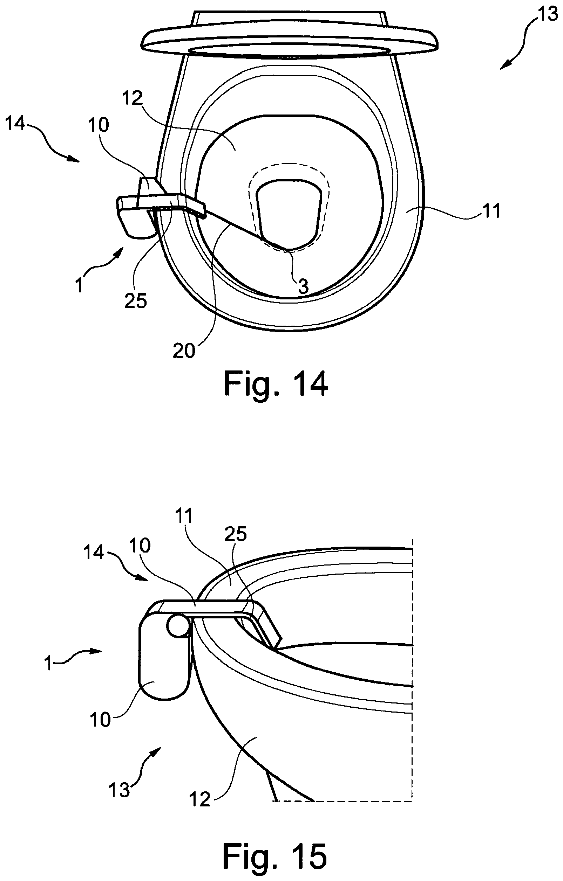

Mittels dieses Haltbügels 10 kann die Vorrichtung 1 beispielsweise an einen Rand 11 einer Toilettenschüssel 12 einer Toilette 13 befestigt werden, wie dies hinsichtlich der entsprechenden Anordnung 14 gemäß den Darstellungen nach den Figuren 14 und 15 gezeigt ist.

Die Entnahmeeinrichtung 2 umfasst im Wesentlichen ein gegenüber dem Gehäuse 6 der Vorrichtung 1 bewegliches Armelement 20 zum Bewegen des Probenträgers 3 in einer axialen Verschieberichtung 21, genauer gesagt, in eine axiale Ausfahrrichtung 22 bzw. in eine axiale Einfahrrichtung 23, ein Führungsteil 25 mit einer Führungsbahn 26 zum präzisen Führen des beweglichen Armelements 20, einen Halter 30 zum Halten eines Probenträgers 3 an dem beweglichen Armelement 20, eine Öffen- und Schließeinheit 32 zum Öffnen und Schließen des Halters 30, eine Löseeinheit 35 zum Lösen eines benutzten Probenträgers 3 aus dem Halter 30, sowie Aktivierungsmittel 38 zum Aktivieren der Beschickungseinrichtung 4 und einer Antriebseinheit 40 zum axialen Treiben des beweglichen Armelements 20.

Die Beschickungseinrichtung 4 umfasst im Wesentlichen einen translatorisch verschieblichen Transportschlitten 42, welcher an einem Führungsbahnteil 44 in einer quer zur axialen Verschieberichtung 21 verlaufenden Zuführrichtung 46 bewegbar ist, einen Aktivierungshebel 48, welcher drehbeweglich an einer Drehachse 50 des Transportschlittens 42 gelagert ist, einer Bewegungskurve 52, an welcher der Aktivierungshebel 48 zusätzlich abrollbar ist, Schneidmittel 54 einer hier nicht näher gezeigten Schneideinrichtung zum Konfektionieren einzelner Probenträger 3, sowie eine zusätzliche Kurvenbahn 56, welche an einen Führungsbolzen 58 des Gehäuses 6 entlanggleitet, um insbesondere die Schneidmittel 54 in Schneidrichtung 60 quer zur Zuführrichtung 46 und entgegen einer Federkraft 62 eines Federelements 64 zu führen, und letztlich ein wieder befüllbares Magazin 66 zur Bevorratung von Rollenmaterial 68, von welchem neue Probenträger 3 mittels der Schneidmittel 54 abgeschnitten werden können.

An dieser Stelle sei noch erwähnt, dass anstelle von Rollenmaterial 68 mit einem entsprechend ausgebildeten Magazin (hier nicht explizit gezeigt) auch andere Bevorratungsausgestaltungen, wie beispielsweise bereits vorkonfektionierte Streifenelemente oder dergleichen als Probenträger, vorgesehen sein können. Eventuell kann hierdurch die Schneideinrichtung 60 weiter vereinfacht werden oder auch zur Gänze entfallen.

Es versteht sich, dass die hier gezeigte Bewegungskurve 52 sowie die zusätzliche Kurvenbahn 56 nur jeweils eine erste Möglichkeit von vielen konstruktiven Möglichkeiten ist, um den Aktivierungshebel 48 oder den Transportschlitten 42 bzw. die Schneideinrichtung wie gewünscht um- und/oder auszulenken. Es könnten stattdessen auch andere Kulissenführungen eingesetzt werden, bei welchen etwa ein Kulissenstein in einer Kulissennut geführt ist usw.

Die Analyseeinrichtung 5 umfasst neben einer Analyseeinheit 70 eine Sensoreinheit 72 mit einer Vielzahl von unterschiedlichen Sensoren 73 (hier nur exemplarisch beziffert) sowie eine Datenübertragungseinheit 74 zum Übertragen von Analysedaten bzw. -informationen an ein nahezu beliebiges Empfängergerät, wie beispielsweise einem Smartphone, auf welchem eine entsprechende Applikation zum Visualisieren und/oder weiter Analysieren der Daten bzw. Informationen läuft.

Hierbei kann die Analyseeinheit 70 aber auch an einer anderen Stelle der Vorrichtung verortet sein, wie beispielsweise in dem Gehäuse 6.

Beispielsweise umfasst die Analyseeinheit 70 zu Analysezwecke einen Microcontroller (nicht gezeigt) oder dergleichen.

Hierbei können die Analyseeinheit 70 und die Sensoreinheit 72 kabelgebunden oder kabellos miteinander in Wirkkontakt stehen. Dies gilt auch im Hinblick auf die vorstehend bereits erwähnte Datenübertragungseinheit 74 oder ähnlichem

Gemäß der Darstellung nach der Figur 1 befindet sich die Vorrichtung 1 in einer Startposition 80, bei welcher das bewegliche Armelement 20 derart in das Führungsteil 25 eingefahren ist, dass sich die Aktivierungsmittel 38 bereits in einer Nut 81 des Aktivierungshebels 48 befinden, wobei jedoch der Aktivierungshebel 48 noch nicht von den Aktivierungsmitteln 38 um die Drehachse 50 gedreht ist oder wenn doch, dann nur vernachlässigbar gering.

Die Aktivierungsmittel 38 sind in diesem Ausführungsbeispiel beispielhaft als Erhebung 82 an der Oberseite des beweglichen Armelements 20 ausgebildet.

Diese Erhebung 82 ist in diesem Ausführungsbeispiel als Stiftelement (nicht nochmals explizit beziffert) ausgebildet.

Es versteht sich, dass bei entsprechender Umgestaltung der Vorrichtung 1 diese Erhebung 82 bzw. das nicht bezifferte Stiftelement auch an der Unterseite 85 des beweglichen Armelements 20 ausgestaltet sein kann.

Jedenfalls befindet sich der Aktivierungshebel 48 in einem noch unaktivierten Zustand und er ist hierbei im Wesentlichen noch senkrecht zu der axialen Verschieberichtung 21 ausgerichtet.

Das Rollenmaterial 68 ist mittels des Transportschlittens 42 bereits unterhalb der Schneidmittel 54 nachgeführt.

Die vorliegenden Schneidmittel 54 können unterschiedlich ausgestaltet sein, beispielsweise als Schneidmesser, Trennmesser oder Stanzmesser, so dass unter dem Begriff Schneidmittel 54 nicht nur das Schneiden, sondern auch Stanzen, Reißen oder sonstige Trennverfahren verstanden werden können.

Der Transportschlitten 42 wird hierbei mittels Federkraft 62 des Blattfederelements 64, welches sich im Magazingehäuse 87 angeordnet befindet, nach oben gedrückt, also in entgegengesetzter Richtung zur eigentlichen Schneidrichtung 60.

Die Schneidmittel 54 sind hierbei bevorzugt mit dem Magazingehäuse 87 auswechselbar, sobald das Rollenmaterial 68 aufgebraucht ist.

Gemäß der Darstellung nach der Figur 2, in welcher im Wesentlichen das Magazin 66 der Vorrichtung 1 gezeigt ist, wird nochmals verdeutlicht, dass der Transportschlitten 42 und das Blattfederelement 64 derart miteinander wechselwirken, dass der Transportschlitten 42 nicht nur durch die Federkräfte 62 entgegen der Schneidrichtung 60 nach oben verlagert wird, sondern darüber hinaus auch in die rückwärtige Richtung 88 weg von dem beweglichen Armelement 20.

Darüber hinaus ist das Magazin 66 unterhalb des Transportschlittens 42 noch mit einem Verschlussteil 89 ausgestattet, welches im Wesentlichen der Bewegung des Transportschlittens 42 folgen kann und bei Inaktivität das Magazingehäuse 87 an der Zuführöffnung 90, aus welcher das Rollenmaterial 68 herausgefördert wird, verschließen kann. Hierdurch kann das Rollenmaterial 68 beispielsweise auch gut vor Luftfeuchtigkeit geschützt werden.

An dem vorderen, freien Ende 87 des beweglichen Armelements 20 ist der Halter 30 zum Halten des jeweiligen Probenträgers 3 angeordnet, wobei der Halter 30 in diesem Ausführungsbeispiel zwei Klemmelemente 88 und 89 aufweist.

Zumindest das zweite Klemmelement 89 ist durch eine Halterbetätigung 90 elastisch auslenkbar, so dass der Halter 30 durch die Halterbetätigung 90 geöffnet werden kann, wodurch dann mittels der Beschickungseinrichtung 4 ein neuer Probenträger 3 in den Halter 30 eingelegt werden kann.

An dieser Stelle sei noch erwähnt, dass gemäß der Darstellung nach der Figur 1 ist das zweite Klemmelement 89 teilweise beschnitten aufgeklappt dargestellt, um das unter dem zweiten Klemmelement 89 verdeckt liegende Rampenelement 91 zu visualisieren.

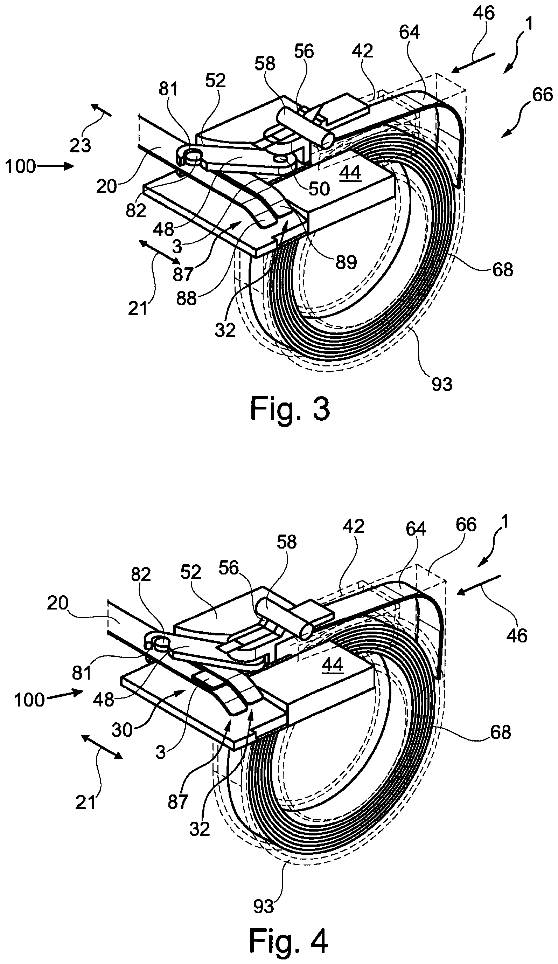

Ferner ist in diesem Ausführungsbeispiel die Halterbetätigung 90 als Rampenelement 91 (vgl. insbesondere Figur 4) ausgestaltet, auf welches das zweite Klemmelement 89 auflaufen kann, so dass es nach oben ausgelenkt wird.

Gemäß der Darstellung nach der Figur 3 ist die Vorrichtung 1 bereits in einer weiter fortgeschrittenen Beschickungsposition 100 dargestellt, bei welcher das bewegliche Armelement 20 weiter in axialer Einfahrrichtung 23 visualisiert ist, so dass der Aktivierungshebel 48 bereits um die Drehachse 50 weitergedreht ist, wobei der Aktivierungshebel 48 sich gleichzeitig an der Bewegungskurve 52 abstützt.

Hierdurch wird der Transportschlitten 42 in Zuführrichtung 46 auf das bewegliche Armelement 20 zubewegt, wodurch der Probenträger 3 nunmehr weiter in den mehr geöffneten Halter 30 eingeschoben werden kann.

Die Bewegung des Transportschlittens 42 in Zuführrichtung 46 auf das bewegliche Armelement 20 zu kann gut daran erkannt werden, dass sich die Kurvenbahn 56 bereits weiter gegenüber dem Führungsbolzen 58 verschoben hat.

Durch das axiale Verschieben des beweglichen Armelements 20 in axialer Einfahrrichtung 23 ist das zweite Klemmelement 89 des Halters 30 weiter nach oben ausgelenkt, da es mit seinem gekrümmten Ende (nicht nochmals beziffert) immer mehr auf das Rampenelement 91 aufläuft.

Gemäß der Darstellung nach der Figur 4 ist die Vorrichtung 1 in einer Schneidposition 110 gezeigt, bei welcher das bewegliche Armelement 20 noch weiter in axialer Einfahrrichtung 23 bewegt ist, so dass der Transportschlitten 42 noch weiter auf das bewegliche Armelement 20 zubewegt ist, die Kurvenbahn 56 wiederum noch weiter unter den Führungsbolzen 58 geführt ist, so dass der Transportschlitten 42 und die daran befestigten Schneidmittel 54 in Schneidrichtung 60 runterwärts bewegt wurden.

Insofern ist nun ein neuer Probenträger 3 von dem in dem Magazin 66 befindlichen Rollenmaterial 68 abgetrennt.

Gut erkennbar ist hierbei auch, dass das zweite Klemmelement 89 nunmehr vollständig auf dem Rampenelement 91 aufgelaufen ist, wodurch der Halter 30 vollständig geöffnet ist und der neue Probenträger 3 somit nun ungehindert weiter von der Beschickungseinrichtung 4 an den Halter 30 übergeben werden kann.

Ist der neue Probenträger 3 letztendlich ordnungsgemäß in dem Halter 30 positioniert, wird das bewegliche Armelement 20 in axiale Ausfahrrichtung 22 bewegt, wodurch das zweite Klemmelement 89 wieder von dem Rampenelement 91 fortbewegt wird, so dass als Konsequenz hieraus sich der Halter 30 schließt und der neue Probenträger 3 in dem Halter 30 fest und betriebssicher geklemmt ist, wobei dies nicht nochmals explizit dargestellt ist.

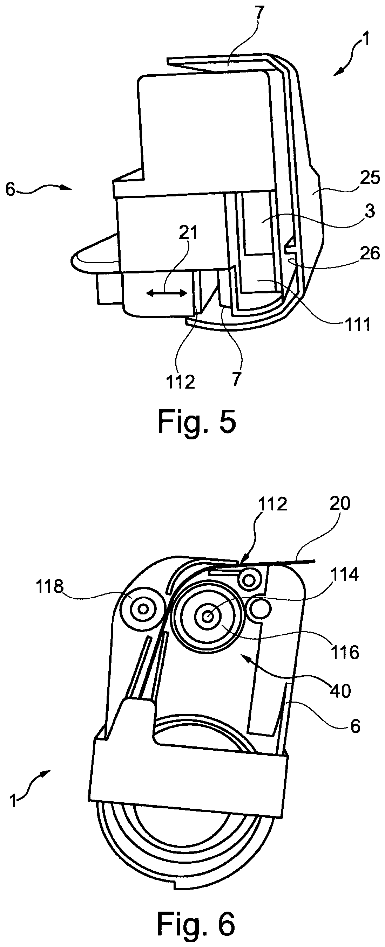

Gemäß der Darstellung nach der Figur 5 ist die Vorrichtung 1 mit ihrem Gehäuse 6 und dem daran angeordneten Haltebügel 10 gezeigt, wobei ein Übergabebereich 111 zwischen der Beschickungseinrichtung 4 und der Entnahmeeinrichtung 2 mit einem dort exemplarisch positionierten Probenträger 3 dargestellt ist. Hierbei ist auf die nochmalige Darstellung des beweglichen Armelements 20 verzichtet worden, wobei dieses bewegliche Armelement 20 normalerweise aus der Austrittsöffnung 112 herausragt.

Gemäß der Darstellung nach der Figur 6 ist dies im Zusammenhang mit der Antriebseinheit 40 der Entnahmeeinrichtung 2 gezeigt, wobei das bewegliche Armelement 20 aufgerollt dargestellt ist und bereits aus der Austrittsöffnung 112 herausgeführt ist.

Von der Antriebseinheit 40 sind stellvertretend nur die Antriebswelle 114 mit der daran angeordneten Antriebswalze 116, sowie die Andrückwalze 118 dargestellt, wobei das bewegliche Armelement 20 zwischen der Antriebswalze 116 und der Andrückwalze 118 hindurchgeführt ist.

Ansonsten liegt das bewegliche Armelement 20 weitestgehend aufgerollt im Gehäuse 6 vor.

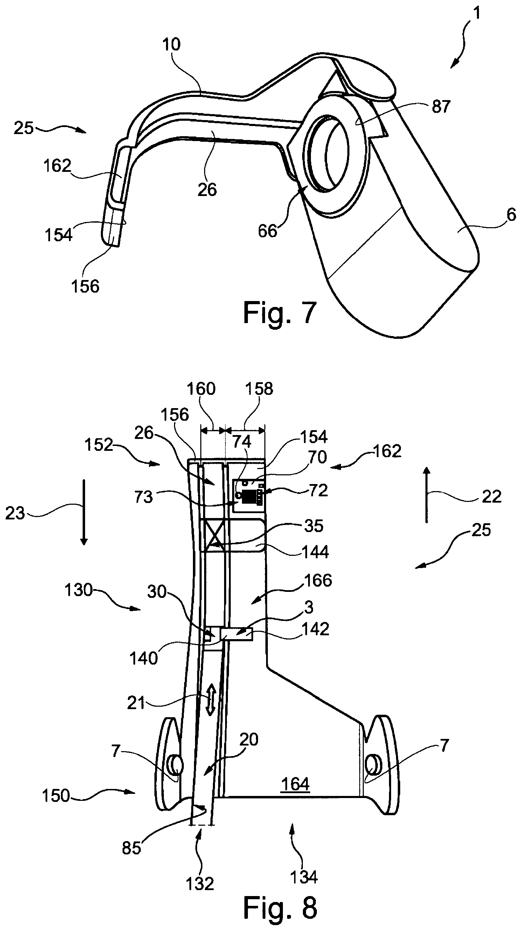

Gemäß der Darstellung nach der Figur 7 ist die Vorrichtung 1 mit abgeklapptem Führungsteil 25 dargestellt, so dass die Vorrichtung 1, wie in den Figuren 14 und 15 gezeigt, an dem Rand 11 der Toilettenschüssel 12 befestigt werden kann.

Vorteilhafterweise ist das Führungsteil 25 vorliegend als Haltebügel 10 ausgestaltet, sodass die Vorrichtung 1 sehr kompakt bauen kann.

Gemäß der Darstellung nach der Figur 8 ist das Führungsteil 25 von seiner Unterseite 130 her detaillierter dargestellt, wobei gut zu erkennen ist, dass sich das Führungsteil 25 einerseits in einen Kontaminationsbereich 132, in welchem die Führungsbahn 26 sich in axialer Verschieberichtung 21 erstreckt, und andererseits einen Reinbereich 134 unterteilt, in welchem zum einen ein neuer Probenträger 3 zumindest teilweise neben der Führungsbahn 26 geführt werden kann und zum anderen insbesondere die Sensoreinheit 72 angeordnet ist.

Hierbei ist der Reinbereich 134 seitlich neben dem Kontaminationsbereich 132 angeordnet, wobei zwischen dem Kontaminationsbereich 132 und dem Reinbereich 134 eine

Trennung 136 vorgesehen ist, die sich in der axialen Verschieberichtung 21 in Längserstreckung des Führungsteils 25 mit einer starren Trennwand 138 erstreckt.

Die Trennwand 138 ist hierbei derart angeordnet und vor allem ausgestaltet, dass sie von einem an dem Halter 30 geklemmten Probenträger 3 überbrückt werden kann.

Mit anderen Worten bedeutet dies, dass sich der an dem Halter 30 gehalterte neue Probenträger 3 mit seiner Klemmseite 140 sich in dem Kontaminationsbereich 132 befindet, während der Probenträger 3 sich mit seiner Sensorseite 142 in dem Reinbereich 134 befindet.

Hierdurch kann sichergestellt werden, dass die Sensorseite 142 eines neuen Probenträgers 3 nicht bereits schon auf seinem Weg in axialer Ausfahrrichtung 22 von Exkrement verunreinigt wird, da ein zuvor benutzter Probenträger 3 lediglich bis auf die axiale Höhe der Sensoreinheit 72 und maximal bis auf die axiale Höhe der Löseeinheit 35 bzw. eines radial daneben angeordneten Übergangsbereichs 144 gelangen kann.

In diesem Ausführungsbeispiel ist das Führungsteil 25 an seiner Unterseite 130 offen, so dass speziell der Kontaminationsbereich 132 und der Reinbereich 134 stets gut gereinigt bzw. desinfiziert werden können.

Hierbei ist der Reinbereich 134, in axialer Einfahrrichtung 23 gesehen, axial hinter der Löseeinheit 35 angeordnet.

Insofern ist der Reinbereich 134, in axialer Ausfahrrichtung 22 gesehen, axial vor der Sensoreinheit 72 angeordnet.

Die Führungsbahn 26 erstreckt sich von dem hinteren Ende 150 des Führungsteils 25 bis zu dem vorderen Ende 152 des Führungsteils 25.

Der Reinbereich 134 erstreckt sich im Wesentlichen von dem hinteren Ende 150 des Führungsteils 20 bis zu dem Übergangsbereich 144, wobei sich an diesen Übergangsbereich 144 die Sensoreinheit 72 an dem vorderen, freien Ende 152 anschließt.

Die Sensoreinheit 72 besitzt an dem vorderen, freien Ende 152 eine Sensorenbereichsöffnung 154 mit einer geringeren Öffnungshöhe (nicht explizit beziffert) als eine Führungsbahnöffnung 156 der Führungsbahn 26.

Hierbei weist die Sensorenbereichsöffnung 154 eine Breite 158 auf, welche größer ist als die Breite 160 der Führungsbahn 26.

Ferner ist der Reinbereich 134 an seiner dem Sensorenbereich 162 abgewandten Seite 164 breiter ausgestaltet, als an seiner dem Sensorenbereich 162 zugewandten Seite 166.

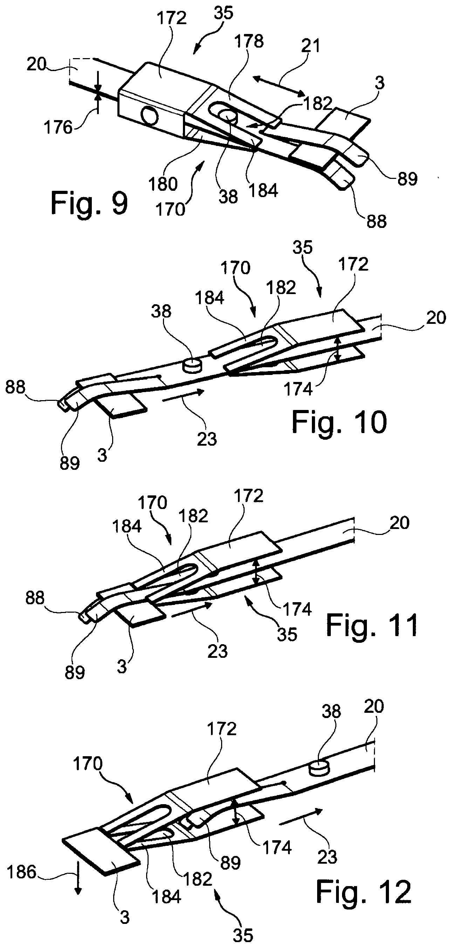

Gemäß den Darstellungen nach den Figuren 9 bis 12 ist die Löseeinheit 35 im Zusammenspiel mit dem Halter 30 des axial beweglichen Armelements nochmal detaillierter illustriert.

Wie aus der Figur 8 bereits ersichtlich, ist die Löseeinheit 35 in dem Übergangsbereich 144 auf axialer Höhe zwischen dem Sensorenbereich 162 und dem Reinbereich 134 angeordnet.

Die Löseeinheit 35 besitzt Abstreifmittel 170, welche dergestalt sind, dass sie nur in der axialen Einfahrrichtung 23 (siehe insbesondere Figuren 10 bis 12) gegenüber dem Probenträger 3 abstreifend bzw. lösend wirken, so dass nur ein benutzter Probenträger 3 aus dem Halter 30 gelöst wird, wenn das axial bewegliche Armelement 20 in axialer Einfahrrichtung 23 durch die Löseeinheit 35 hindurch bewegt wird.

Hierzu weist die Löseeinheit 35 einen von dem axial beweglichen Armelement 20 durchdringbaren Grundkörper 172 auf.

Der Grundkörper 172 besitzt hierbei eine Raumhöhe 174, welche in diesem Ausführungsbeispiel mehr als 10 mm beträgt.

Jedenfalls ist die Raumhöhe 174 um ein Vielfaches größer als die Armelementdicke 176.

Die Abstreifmittel 170 der Löseeinheit 35 sind in diesem Ausführungsbeispiel als ein erstes Fingerelement 178 an der Oberseite 84 des axial beweglichen Armelements 20 und als unteres flexibles Fingerelement 180 an der Unterseite 85 des axial beweglichen Armelements 20 ausgebildet.

Beide flexiblen Fingerelemente 178 und 180 sind zweigeteilt.

Das heißt, jedes der flexiblen Fingerelemente 178, 180 weist zwei durch eine Lücke 182 voneinander beabstandete flexible Fingerteile 184 (nur exemplarisch beziffert) auf.

Die Lücke 182 ist hierbei derart gewählt, dass durch sie problemlos die Aktivierungsmittel 38 hindurchfahren können, so dass die Aktivierungsmittel 38 die Aufgabe der Löseeinheit 35 nicht beeinflussen, sondern nur zur Aktivierung der bereits vorstehend ausführlich beschriebenen Beschickungseinrichtung 4 dienen.

Darüber hinaus ist es vorteilhaft, dass die flexiblen Fingerteile 184 sich vorhandenen Konturierungen des axial beweglichen Armelements 20 anpassen bzw. diesen flexibel folgen können, so dass auch der eigentliche Halter 30, der an dem axial beweglichen Armelement 20 ausgestaltet ist, diese Löseeinheit 35 problemlos passieren kann.

Insofern wechselwirkt die Löseeinheit 35 im Sinne der Erfindung nur mit einem benutzten Probenträger 3, so dass dieser aus dem Halter 30 des axial beweglichen Armelements 20 herausgelöst wird, wenn das axial bewegliche Armelement 20 in axialer Einfahrrichtung 23 mit dem Halter 30 durch die Löseeinheit 35 hindurch verlagert wird.

Nur dann wird der benutzte Probenträger 3 mittels der flexiblen Fingerteile 184 aus dem Halter 30 herausgelöst und der benutzte Proebenträger 3 fällt gemäß der Entsorgungsrichtung 186 (siehe Figur 12) in die Toilettenschüssel 12 (siehe Figuren 14 und 15), so dass er über die Toilette 13 einfach entsorgt werden kann.

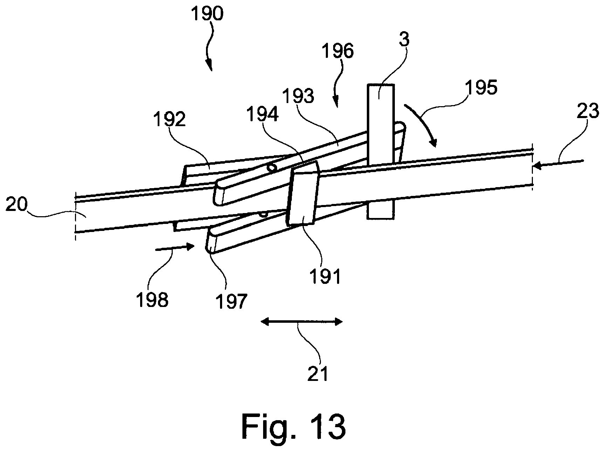

Der in der Figur 13 gezeigte alternative Halter 190 weist eine Befestigung 191 mit einem Befestigungskörper 192 auf, mittels welcher der Halter 190 an dem beweglichen Armelement 20 festgelegt werden kann. Hierzu genügt es, den Halter 190 mit einer Montagekraft einfach auf das bewegliche Armelement 20 aufzuschieben, wobei die Montagekraft wesentlich höher ist, als alle übrigen im normalen Betrieb der Vorrichtung 1 auf den Halter 190 wirkenden Arbeitskräfte. Hierdurch sitzt der Halter 190 betriebssicher auf dem beweglichen Armeelement 20.

Der Halter 190 verfügt über ein Klemmelement 193, welches mittels einer federvorgespannten Rotationsachse 194 drehbar an dem Befestigungskörper 192 gelagert ist.

Hierbei funktioniert der Halter 190 im Wesentlichen ähnlich einem federvorgespannten Klappmechanismus.

Grundsätzlich wird das Klemmelement 193 durch die Federkräfte der federvorgespannten Rotationsachse 194 in Klemmrichtung 195 bewegt, so dass ein Probenträger 3 betriebssicher zwischen dem beweglichen Armelement 20 und dem Klemmelement 193 eingeklemmt ist.

Geöffnet wird der Halter 190, indem das bewegliche Armelement 20 in Einfahrrichtung 23 bewegt wird und das der Klemmseite 196 abgewandte Ende 197 des Klemmelements 193 gegen einen Widerstand (hier nicht gezeigt) gedrückt wird, wodurch auf dieses Ende 197 eine Öffenkraft 198 wirkt.

Dieser Widerstand befindet sich normalerweise an der Beschickungseinrichtung 4, da dort der Halter 190 zum Aufnehmen eines neuen Probenträgers 3 auch geöffnet werden soll.

Der Widerstand kann durch eine mechanische Zwangsführung oder sonstige mechanische Elemente verwirklicht sein, welche entlang des beweglichen Armelements 20 bevorzugt einstellbar angeordnet sein können. Hierdurch kann die Position eindeutig bestimmt werden, an welcher der Halter 190 letztendlich geöffnet wird, um einen neuen Probenträger 3 in Empfang zu nehmen, oder aber auch um einen benutzten Probenträger 3 loszulassen und von dem Halter 190 zu lösen.

Gemäß der Darstellung nach der Figur 14 ist die vorliegende Vorrichtung 1 an dem Rand 11 der Toilettenschüssel 12 aufgehängt. Hierbei ist das axial bewegliche Armelement 20 insbesondere aus dem Führungsteil 25 heraus bereits in die Toilettenschüssel 12 hinein ausgefahren, wobei somit ein neuer Probenträger 3 derart innerhalb der Toilettenschüssel 12 platziert ist, dass der Probenträger 3 zur Aufnahme insbesondere von Urin gut an der Toilette 13 platziert ist.

Gemäß der weiteren Darstellung nach der Figur 15 ist nochmals etwas detaillierter gezeigt, wie die Vorrichtung 1 mittels des Haltebügels 10 an der Toilettenschüssel 12 platziert ist.

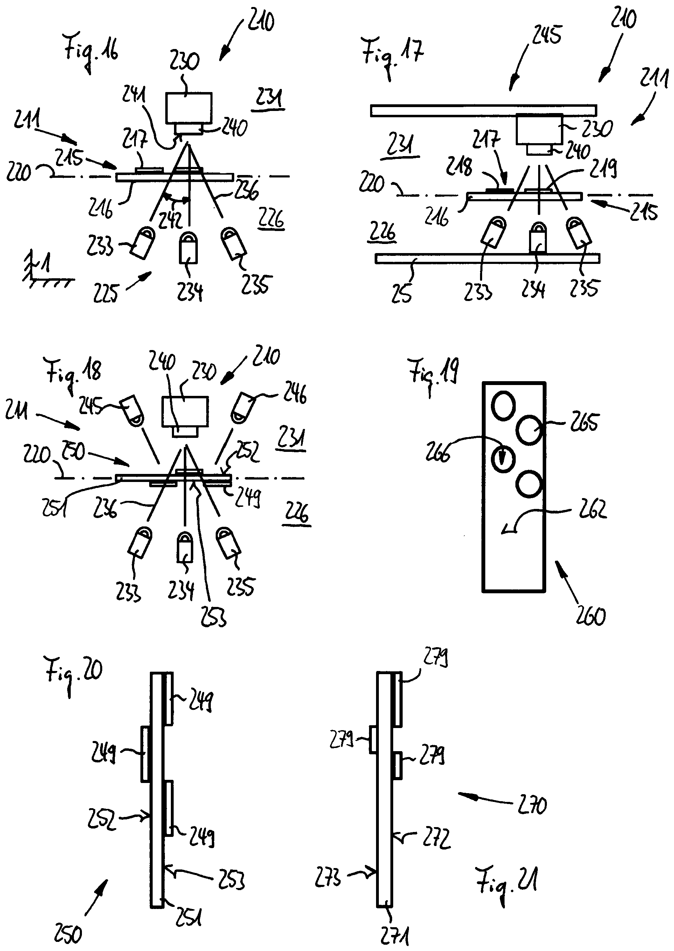

Gemäß den Darstellungen nach den Figuren 16 und 17 ist des Weiteren noch eine andere Sensoreinheit 210 mit einer Durchlichtapparatur 211 gezeigt, wobei insbesondere auch die vorstehend beschriebene Vorrichtung 1 mit dieser anderen Sensoreinheit 210 kumulativ oder alternativ ausgerüstet werden kann.

Mittels dieser anderen Sensoreinheit 210 gelingt es, einen Probenträger 215 nicht nur von einer Seite zu beleuchten, etwa im Sinne einer Reflexionsphotometrie, sondern es gelingt darüber hinaus, einen entsprechend ausgestalteten Probenträger 215 zur Gänze oder teilweise auch zu durchleuchten.

Hierzu weist der Probenträger 215 einen lichtdurchlässigen Grundkörper 216 sowie lichtdurchlässige Probenanalysefelder 217 (nur exemplarisch beziffert) auf, wobei diese lichtdurchlässigen Probenanalysefelder 217 auch mit unterschiedlichen Indikatoren 218 und 219 für verschiedene Analysen ausgestattet sein können (nur beispielhaft beziffert, vgl. Figuren 17 und 21).

Die Durchlichtmessapparatur 211 ist zumindest teilweise beidseits des Probenträgers 215 angeordnet.

Genauer gesagt ist die Durchlichtmessapparatur 211 zumindest teilweise beidseits einer Transportstrecke 220 angeordnet, wobei die Transportstrecke 220 ein integraler Bestandteil der Durchlichtmessapparatur 211 ist, entlang welcher der Probenträger 215 bewegbar ist.

Insbesondere weist die Durchlichtapparatur 211 in diesem Ausführungsbeispiel eine Beleuchtungseinrichtung 225 auf einer ersten Seite 226 der integralen Transportstrecke 220 sowie eine Detektiereinrichtung 230 auf einer der ersten Seite 226 gegenüberliegenden, zweiten Seite 231 der integralen Transportstrecke 220 auf.

Die Beleuchtungseinrichtung 225 besitzt hierbei drei Lichtquellen 233, 234 und 235 mit jeweiligen Hauptstrahlengängen 236 (nur exemplarisch beziffert), wobei die Lichtquellen 233, 234 und 235 hier als LED ausgeführt sind.

Die Detektiereinrichtung 230 zeichnet sich durch wenigstens eine Detektiersensorfläche 240 aus, welche in diesem Ausführungsbeispiel mit Ihrer Detektierseite 241 den Lichtquellen 233, 234 und 235 zugewandt angeordnet ist.

Die Durchlichtmessapparatur 211 weist somit mehr als eine Lichtquelle 233, 234, 235 auf, wobei der Hauptstrahlengang 236 der Lichtquellen 234 rechtwinkelig zu der Detektiersensorfläche 240 und die Hauptstrahlengänge 236 der weiteren Lichtquellen 233 und 235 in einem hiervon abweichenden Winkel 242 zu der Detektiersensorfläche 240 verlaufend angeordnet sind.

Die andere Sensoreinheit 210 kann mit einfachen Gehäuse 245 zumindest teilweise bestehend aus dem Führungsteil 25 der Vorrichtung ausgestattet sein, wie dies in der Figur 17 zu erkennen ist.

Gemäß der Darstellung nach der Figur 18 ist die in den Figuren 16 und 17 gezeigte Sensoreinheit 210 mit der Durchlichtmessapparatur 211 ausgestattet und besitzt darüber hinaus noch zwei zusätzliche Lichtquellen 245 und 246, welche auf derselben Seite der integralen Transportstrecke 220 angeordnet sind, wie die Detektiereinrichtung 230, nämlich der zweiten Seite 231.

Mittels der zwei zusätzlichen Lichtquellen 245 und 246 können Probenanalysefelder 249 des in die Sensoreinheit 210 eingebrachten alternativen Probenträgers 250 entweder zusätzlich durchleuchtet oder reflektierend beleuchtend werden.

An dem alternativen Probenträger 250 sind die Probenanalysefelder 249 beidseits an seinem Grundkörper 251 (nur exemplarisch beziffert) angeordnet, und zwar an seiner Vorderseite 252 ein einzelnes Probenanalysefeld 249 und an seiner Rückseite 253 zwei Probenanalysefelder 249, so dass bei gleicher Probenträgergröße mehr Probenanalysefelder 249 an dem alternativen Probenträger 250 untergebracht werden können.

Gemäß der Darstellung nach der Figur 19 ist nochmals ein anderer Probenträger 260 gezeigt, an dessen Vorderseite 262 unterschiedlich große, runde Probenanalysefelder 265 (nur exemplarisch beziffert) mit unterschiedlichen Indikatormaterialien 266 (nur exemplarisch beziffert) platziert sind.

Die Figur 20 zeigt den Probenträger 250 aus der Figur 18 in einer Seitenansicht.

Einen weiteren alternativen Probenträger 270 zeigt die Figur 21. Der weitere alternative Probenträger 270 weist einen Grundkörper 271 auf, an dessen Vorderseite 272 zwei Probenanalysefelder 279 unterschiedlicher Größe und an dessen Rückseite 273 ein einzelnes Probenanalysefeld 279 platziert sind.

An dieser Stelle sei explizit darauf hingewiesen, dass Merkmale der vorstehend bzw. in den Ansprüchen und/oder Figuren beschriebenen Lösungen gegebenenfalls auch kombiniert werden können, um die erläuterten Merkmale, Effekte und Vorteile entsprechend kumuliert umsetzen bzw. erzielen zu können.

Es versteht sich, dass es sich bei dem vorstehend erläuterten Ausführungsbeispiel lediglich um eine erste Ausgestaltung der erfindungsgemäßen Vorrichtung handelt. Insofern beschränkt sich die Ausgestaltung der Erfindung nicht auf dieses Ausführungsbeispiel.

Sämtliche in den Anmeldungsunterlagen offenbarten Merkmale werden als erfindungswesentlich beansprucht, sofern sie einzeln oder in Kombination miteinander gegenüber dem Stand der Technik neu sind.

### Liste der verwendeten Bezugszeichen

- 1: Vorrichtung zur Vor-Ort-Analyse von Exkrementen
- 2: Entnahmeeinrichtung
- 3: Probenträger (neu oder benutzt)
- 4: Beschickungseinrichtung
- 5: Analyseeinrichtung
- 6: Gehäuse
- 7: Gelenkverbindung
- 10: Haltebügel
- 11: Rand
- 12: Toilettenschüssel
- 13: Toilette
- 14: Anordnung
- 20: axial bewegliches Armelement
- 21: axiale Verschieberichtung
- 22: axiale Ausfahrrichtung
- 23: axiale Einfahrrichtung
- 25: Führungsteil
- 26: Führungsbahn
- 30: Halter
- 32: Offen- und Schließeinheit
- 35: Löseeinheit
- 38: Aktivierungsmittel
- 40: Antriebseinheit
- 42: Transportschlitten
- 44: Führungsbahnteil
- 46: Zuführrichtung
- 48: Aktivierungshebel
- 50: Drehachse
- 52: Bewegungskurve
- 54: Schneidmittel
- 56: Kurvenbahn
- 58: Führungsbahn
- 60: Schneidrichtung
- 62: Federkräfte
- 64: Blattfederelement
- 66: Magazin
- 68: Rollenmaterial
- 70: Analyseeinheit
- 72: Sensoreinheit
- 73: Sensoren (gleiche oder verschiedene)
- 74: Datenübertragungseinheit
- 80: Startposition
- 81: Nut
- 82: Erhebung
- 84: Oberseite
- 85: Unterseite
- 87: vorderes, freies Ende
- 88: erstes Klemmelement
- 89: zweites Klemmelement
- 90: Halterbetätigung
- 91: Rampenelement
- 93: Magazingehäuse
- 94: rückwärtige Richtung
- 95: Verschlussteil
- 96: Zuführöffnung
- 100: Beschickungsposition
- 110: Schneidposition
- 111: Übergabebereich
- 112: Austrittsöffnung
- 114: Antriebswelle
- 116: Antriebswalze
- 118: Andrückwalze
- 130: Unterseite
- 132: Kontaminationsbereich
- 134: Reinbereich
- 136: Trennung
- 138: Trennwand
- 140: Klemmseite
- 142: Sensorseite
- 144: Übergangsbereich
- 150: hinteres Ende
- 152: vorderes, freies Ende
- 154: Sensorenbereichsöffnung
- 156: Führungsbahnöffnung
- 158: Breite
- 160: Breite
- 162: Sensorenbereich
- 164: abgewandte Seite
- 166: zugewandte Seite
- 170: Abstreifmittel
- 172: Grundkörper
- 174: Raumhöhe
- 176: Armelementedicke
- 178: oberes Fingerelemente
- 180: unteres Fingerelement
- 182: Lücke
- 184: flexible Fingerteile
- 186: Entsorgungsrichtung
- 190: alternativer Halter
- 191: Befestigung
- 192: Befestigungskörper
- 193: Klemmelement
- 194: federvorgespannte Rotationsachse
- 195: Klemmrichtung
- 196: Klemmseite
- 197: abgewandtes Ende
- 198: Öffenkraft

- 210: andere Sensoreinheit
- 211: Durchlichtmessapparatur
- 215: Probenträger
- 216: Grundkörper
- 217: Probenanalysefelder
- 218: ein erster Indikator
- 219: ein weiterer Indikator
- 220: Transportstrecke
- 225: Beleuchtungseinrichtung
- 226: erste Seite
- 230: Detektiereinrichtung
- 231: zweite Seite
- 233: erste Lichtquelle
- 234: zweite Lichtquelle
- 235: dritte Lichtquelle
- 236: Hauptstrahlengänge
- 240: Detektiersensorfläche
- 241: Detektierseite
- 242: Winkel
- 245: erste zusätzliche Lichtquelle
- 246: zweite zusätzliche Lichtquelle

- 249: Probenanalysefelder
- 250: alternativer Probenträger
- 251: Grundkörper
- 252: Vorderseite
- 253: Rückseite

- 260: anderer Probenträger
- 262: Vorderseite
- 265: runde Probenanalysefelder
- 266: unterschiedliche Indikatormaterialien

- 270: weiterer alternativer Probenträger
- 271: Grundkörper
- 272: Vorderseite
- 273: Rückseite
- 279: Probenanalysefelder

## Patentansprüche

1. Vorrichtung (1) zur Vor-Ort-Analyse von Exkrementen mit einem Gehäuse (6), mit einer Entnahmeeinrichtung (2), mittels welcher eine Probe an Exkrement entnehmbar ist, bei welcher die Entnahmeeinrichtung (2) ein gegenüber dem Gehäuse (6) in axialer Verschieberichtung (21) bewegliches Armelement (20) mit einem Halter (30) für einen Probenträger (3; 215; 250; 260; 270) umfasst, welches zumindest teilweise in einem Führungsteil (25) ein- und ausfahrbar geführt ist, mit einer Beschickungseinrichtung (4), mittels welcher ein neuer Probenträger (3; 215; 250; 260; 270) für die Entnahmeeinrichtung (2) bereitstellbar ist, und mit einer Analyseeinrichtung (5), mittels welcher die entnommene Probe zumindest teilweise analysierbar ist, ***dadurch gekennzeichnet, dass*** das Führungsteil (25) einen Kontaminationsbereich (132) mit einer Führungsbahn (26) zum Führen des beweglichen Armeelements (20) und darüber hinaus einen Reinbereich (134) aufweist, in welchem ein neuer Probenträger (3; 215; 250; 260; 270) neben der Führungsbahn (26) anordenbar ist und von der Beschickungseinrichtung (4) an einen an dem Armelement (20) befindlichen Halter (30) übergeben wird.

2. Vorrichtung (1) gemäß Anspruch 1, ***gekennzeichnet durch*** eine Sensoreinheit (71; 210) mit einer Durchlichtmessapparatur (211) zum Durchleuchten des Probenträgers (3; 215; 250; 260; 270).

3. Vorrichtung (1) nach Anspruch 2, ***dadurch gekennzeichnet, dass*** die Durchlichtmessapparatur (211) zumindest teilweise beidseits des Probenträgers (3; 215; 250; 260; 270) angeordnet ist.

4. Vorrichtung (1) nach Anspruch 2 oder 3, ***dadurch gekennzeichnet, dass*** die Durchlichtmessapparatur (211) eine integrale Transportstrecke (220) aufweist, entlang welcher der Probenträger (3; 215; 250; 260; 270) bewegbar ist.

5. Vorrichtung (1) nach Anspruch 4, ***dadurch gekennzeichnet, dass*** die Durchlichtmessapparatur (211) eine Beleuchtungseinrichtung (225) auf einer ersten Seite (226) der integralen Transportstrecke (220) und eine Detektiereinrichtung (230) auf einer der ersten Seite (226) gegenüberliegenden, zweiten Seite (231) der integralen Transportstrecke (220) angeordnet ist.

6. Vorrichtung (1) nach einem der Ansprüche 2 bis 5, ***dadurch gekennzeichnet, dass*** die Durchlichtmessapparatur (211) mehr als eine Lichtquelle (233, 234, 235; 245, 246) aufweist, wobei ein Hauptstrahlengang (236) wenigstens einer der Lichtquellen (233, 234, 235; 245, 246) rechtwinkelig zu einer Detektiersensorfläche (240) und wenigstens ein weiterer Hauptstrahlengang (236) wenigstens einer weiteren der Lichtquellen (233, 234, 235; 245, 246) in einem hiervon abweichenden Winkel (242) zu einer Detektiersensorfläche (240) verlaufend angeordnet sind.

7. Vorrichtung (1) nach einem der Ansprüche 1 bis 6, ***dadurch gekennzeichnet, dass*** eine Sensoreinheit (72; 210) in axialer Einfahrrichtung (23) des beweglichen Armelements (20) gesehen axial vor einer Löseeinheit (35) zum Lösen eines benutzten Probenträgers (3; 215; 250; 260; 270) von dem beweglichen Armelement (20) angeordnet ist.

8. Vorrichtung (1) nach einem der Ansprüche 1 bis 6 , ***dadurch gekennzeichnet, dass*** die Entnahmeeinrichtung (2) eine Löseeinheit (35) mit Abstreifmitteln (170) zum Lösen eines benutzten Probenträgers (3; 215; 250; 260; 270) von dem beweglichen Armelement (20) umfasst.

9. Vorrichtung (1) nach einem der Ansprüche 1 bis 7, ***gekennzeichnet durch*** eine Sensoreinheit (72; 210) mit einem Thermosensor zum Detektieren einer Exkrementtemperatur, einer Körpertemperatur, eines Exkrementvolumens und/oder eines Exkrementvolumenstroms.

10. Verfahren zum Betreiben einer Vorrichtung (1) zur Vor-Ort-Analyse von Exkrementen, bei welchem ein Probenträger (3; 215; 250; 260; 270) mittels eines axial beweglichen Armelements (20) für eine Probenentnahme gegenüber einer Sensoreinheit (72; 210) in dem Führungsteil (25) linear verfahren wird, bei welchem der Probenträger (3; 215; 250; 260; 270) an einem Halter (30) des axial beweglichen Armelements (20) gehaltert wird und bei welchem eine von dem Probenträger (3; 215; 250; 260; 270) getragene Probe an Exkrement an der Vorrichtung (1) zumindest teilweise analysiert wird, ***dadurch gekennzeichnet, dass*** das Führungsteil (25) einen Kontaminationsbereich (132) mit einer Führungsbahn (26) zum Führen des beweglichen Armeelements (20) und darüber hinaus einen Reinbereich (134) aufweist, in welchem ein neuer Probenträger (3; 215; 250; 260; 270) neben der Führungsbahn (26) angeordnet wird und von der Beschickungseinrichtung (4) an einen an dem Armelement (20) befindlichen Halter (30) übergeben wird.

11. Verfahren nach Anspruch 10, ***dadurch gekennzeichnet, dass*** der benutzter Probenträger (3; 215; 250; 260; 270) aus dem Halter (30) mittels der in axialer Verschieberichtung (21) erfolgten Axialbewegung des Armelements (20) gelöst wird.

12. Verfahren nach Anspruch 10 oder 11, ***dadurch gekennzeichnet, dass*** ein mit Exkrement versehener Probenträger (3; 215; 250; 260; 270) zwischen einer Beleuchtungseinrichtung (225) und einer Detektiereinrichtung (230) verbracht und für eine Durchlichtmessung mittels der Beleuchtungseinrichtung (225) durchleuchtet wird.

13. Verfahren nach Anspruch 12, ***dadurch gekennzeichnet, dass*** der mit Exkrement versehene Probenträger (3; 215; 250; 260; 270) während der Durchlichtmessung ruht oder bewegt wird.

14. Verfahren nach Anspruch 12 oder 13, ***dadurch gekennzeichnet, dass*** eine Benetzung des Probenträgers (3; 215; 250; 260; 270) mit Exkrement, insbesondere mit Urin, mittels zusätzlicher Bewegungen, insbesondere in axialer Aus- und/oder Einfahrrichtung (22, 23), des axial beweglichen Armelements (20) unterstützt wird.

15. Verfahren nach einem der Ansprüche 10 bis 14, ***dadurch gekennzeichnet, dass*** überschüssiges Exkrement, insbesondere überschüssiger Urin, durch Vibration des axial beweglichen Armelements (20) von diesem beweglichen Armelement (20), dem Probenträger (3; 215; 250; 260; 270) und/oder dem Halter (30) entfernt wird.

16. Anordnung (14) bestehend aus einer Toilette (13), einem Urinal oder dergleichen und aus einer Vorrichtung (1) nach einem der Ansprüche 1 bis 9.

17. Vorrichtung nach einem der Ansprüche 1 bis 9, **dadurch gekennzeichnet, dass** der Probenträger (3; 215; 250; 260; 270) für eine Vor-Ort-Analyse von Exkrementen mit einem Grundkörper (216; 251; 271) versehen ist, wobei der Probenkörper (3; 215; 250; 260; 270) geometrisch unterschiedlich geformte, einzelne Probenanalysefelder (217; 249; 265; 279) aufweist.

18. Vorrichtung nach Anspruch 17, **dadurch gekennzeichnet, dass** bei dem Probenträger (3; 215; 250; 260; 270) sowohl an der Vorderseite (252; 262; 272) als auch an der Rückseite (253; 273) des Grundkörpers (216; 251; 271) Probenanalysefelder 217; 249; 265; 279) angeordnet sind.

19. Vorrichtung nach einem der Ansprüche 17 oder 18, ***dadurch gekennzeichnet, dass*** bei dem Probenträger (3; 215; 250; 260; 270) einzelne Probenanalysefelder 217; 249; 265; 279) unterschiedliche Indikatoren (218, 219) aufweisen.

20. Vorrichtung nach einem der Ansprüche 17 bis 19, ***dadurch gekennzeichnet, dass*** bei dem Probenträger (3; 215; 250; 260; 270) einzelne Probenanalysefelder 217; 249; 265; 279) beanstandet voneinander an dem Grundkörper (216; 251; 271) angeordnet sind.

21. Vorrichtung nach einem der Ansprüche 17 bis 20, ***dadurch gekennzeichnet, dass*** bei dem Probenträger (3; 215; 250; 260; 270) der Grundkörper (216; 251; 271) und einzelne Probenanalysefelder 217; 249; 265; 279) zumindest teilweise lichtdurchlässig sind.

## Claims

1. Device (1) for on-site analysis of excrements, having a housing (6) with a sampling device (2) by means of which a sample of excrement can be taken, the sampling device (2) comprising an arm element (20) movable in the axial direction (21) of displacement with respect to the housing (6), the arm element (20) having a support (30) for a sample holder (3; 215; 250; 260; 270) and being guided so as to be at least partially insertable and extractable in a guiding piece (25), having a feeding unit (4) by means of which a new sample holder (3; 215; 250; 260; 270) for the sampling device (2) can be provided, and having an analyzing unit (5) by means of which the taken sample can be at least partially analyzed, ***characterized in that*** the guiding piece (25) has a contamination area (132) with a guiding path (26) for guiding the movable arm element (20) and in addition a clean area (134) in which a new sample holder (3; 215; 250; 260; 270) can be arranged next to the guiding path (26) and is transferred by the feeding unit (4) to a holder (30) located on the arm element (20).

2. Device (1) according to Claim 1, ***characterized by*** a sensor unit (71; 210) having a transmitted-light measuring apparatus (211) for transmitting light through the sample holder (3; 215; 250; 260; 270).

3. Device (1) according to Claim 2, ***characterized in that*** the transmitted-light measuring apparatus (211) is at least partly arranged on both sides of the sample holder (3; 215; 250; 260; 270).

4. Device (1) according to Claim 2 or 3, ***characterized in that*** the transmitted-light measuring apparatus (211) has an integral transport path (220) along which the sample holder (3; 215; 250; 260; 270) is movable.

5. Device (1) according to Claim 4, ***characterized in that*** the transmitted-light measuring apparatus (211) has an illumination device (225) on a first side (226) of the integral transport path (220) and a detection device (230) on a second side (231) of the integral transport path (220) opposite the first side (226).

6. Device (1) according to one of Claims 2 through 5, ***characterized in that*** the transmitted-light measuring apparatus (211) has more than one light source (233, 234, 235; 245, 246), a main optical path (236) of at least one of the light sources (233, 234, 235; 245, 246) being arranged perpendicular to a detection sensor surface (240) and at least one other main optical path (236) of at least one other light source (233, 234, 235; 245, 246) being arranged at an angle (242) to a detection sensor surface (240) which deviates therefrom.

7. Device (1) according to one of Claims 1 through 6, ***characterized in that*** a sensor unit (72; 210) is arranged before a release unit (35), seen in the axial feeding direction (23) of the movable arm element (20), for releasing a used sample holder (3; 215; 250; 260; 270) from the movable arm element (20).

8. Device (1) according to one of Claims 1 through 6, ***characterized in that*** the sampling device (2) comprises a release unit (35) with stripping means (170) for releasing a used sample holder (3; 215; 250; 260; 270) from the movable arm element (20).

9. Device (1) according to one of Claims 1 through 7, ***characterized by*** a sensor unit (72; 210) having a thermal sensor for detecting an excrement temperature, a body temperature, an excrement volume and/or an excrement volume flow.

10. Method of operating a device (1) for on-site analysis of excrements, wherein a sample holder (3; 215; 250; 260; 270) is linearly displaced with respect to a sensor unit (72; 210) in the guiding piece (25) by means of an axially movable arm element; wherein the sample holder (3; 215; 250; 260; 270) is supported at a holder (30) of the axially movable arm element (20) and wherein a sample of excrement carried by the sample holder (3; 215; 250; 260; 270) is at least partially analyzed at the device (1); ***characterized in that*** the guiding piece (25) has a contamination area (132) with a guiding path (26) for guiding the movable arm element (20) and in addition a clean area (134) in which a new sample holder (3; 215; 250; 260; 270) is arranged next to the guiding path (26) and transferred by the feeding unit (4) to a holder (30) positioned on the arm element (20).

11. Method according to Claim 10, ***characterized in that*** the used sample holder (3; 215; 250; 260; 270) is released from the holder (30) by the axial movement of the arm element (20) which takes place in the axial direction (21) of displacement.

12. Method according to Claim 10 or 11, ***characterized in that*** a sample holder (3; 215; 250; 260; 270) with excrement is positioned between an illumination device (225) and a detection device (230) and that light is transmitted through it by the illumination device (225) for transmitted-light measurement.

13. Method according to Claim 12, ***characterized in that*** the sample holder (3; 215; 250; 260; 270) with excrement is at rest or moving during transmitted-light measurement.

14. Method according to Claim 12 or 13, ***characterized in that*** moistening of the sample holder (3; 215; 250; 260; 270) with excrement, in particular with urine, is supported by additional movements, in particular in the axial extension and/or retraction directions (22, 23), of the axially movable arm element (20).

15. Method according to one of Claims 10 through 14, ***characterized in that*** excess excrement, in particular excess urine, is removed from this movable arm element (20), the sample holder (3; 215; 250; 260; 270) and/or the holder (30) by vibration of the axially movable arm element (20).

16. Assembly (14), consisting of a toilet (13), a urinal or the like and of a device (1) according to one of Claims 1 through 9.

17. Device according to one of Claims 1 through 9, ***characterized in that*** the sample holder (3; 215; 250; 260; 270) is provided with a base body (216; 251; 271) for an on-site analysis of excrements, the sample holder (3; 215; 250; 260; 270) having individual sample analysis pads (217; 249; 265; 279) with different geometrical shapes.

18. Device according to Claim 17, ***characterized in that*** both at the front (252; 262; 272) and at the back (253; 273) of the base body (216; 251; 271) of the sample holder (3; 215; 250; 260; 270), sample analysis pads (217; 249; 265; 279) are arranged.

19. Device according to one of Claims 17 or 18, ***characterized in that*** individual sample analysis pads (217; 249; 265; 279) of the sample holder (3; 215; 250; 260; 270) have different indicators (218, 219).

20. Device according to one of Claims 17 through 19, ***characterized in that*** individual sample analysis pads (217; 249; 265; 279) of the sample holder (3; 215; 250; 260; 270) are arranged mutually spaced on the base body (216; 251; 271) .

21. Device according to one of Claims 17 through 20, ***characterized in that*** the base body (216; 251; 271) and individual sample analysis pads (217; 249; 265; 279) of the sample holder (3; 215; 250; 260; 270) are at least partially translucent.

## Revendications

1. Dispositif (1) d'analyse sur place d'excréments, comportant un boîtier (6) doté d'un dispositif de prélèvement (2) au moyen duquel un échantillon d'excréments peut être prélevé et dans lequel le dispositif de prélèvement (2) comprend un élément bras (20) mobile par rapport au boîtier (6) dans le sens de déplacement axial (21) et doté d'un support (30) pour un porte-échantillon (3; 215; 250; 260; 270), lequel élément bras peut être rentré ou sorti au moins partiellement dans ou d'une pièce de guidage (25), un dispositif de transport (4) au moyen duquel un nouveau porte-échantillon (3; 215; 250; 260; 270) pour le dispositif de prélèvement (2) peut être mis à disposition, et un dispositif d'analyse (5) au moyen duquel l'échantillon prélevé est analysable du moins partiellement, **caractérisé en ce que** la pièce de guidage (25) présente une zone de contamination (132) dotée d'une piste de guidage (26) pour le guidage de l'élément bras mobile (20) et en outre une zone blanche (134) dans laquelle un nouveau porte-échantillon (3; 215; 250; 260; 270) peut être disposé près de la piste de guidage (26) et est transféré par le dispositif de transport (4) vers un support (30) se trouvant sur l'élément bras (20).

2. Dispositif (1) selon la revendication 1, **caractérisé par** une unité de détection (71; 210) comportant un appareil de mesure de lumière transmise (211) pour éclairer en transparence le porte-échantillon (3; 215; 250; 260; 270).

3. Dispositif (1) selon la revendication 2, **caractérisé en ce que** l'appareil de mesure de lumière transmise (211) est disposé au moins partiellement des deux côtés du porte-échantillon (3; 215; 250; 260; 270).

4. Dispositif (1) selon la revendication 2 ou 3, **caractérisé en ce que** l'appareil de mesure de lumière transmise (211) comporte un parcours de transport intégral (220) le long duquel le porte-échantillon (3; 215; 250; 260; 270) est mobile.

5. Dispositif (1) selon la revendication 4, **caractérisé en ce que** l'appareil de mesure de lumière transmise (211) comporte un dispositif d'éclairage (225) sur un premier côté (226) du parcours de transport intégral (220) et qu'un dispositif de détection (230) est disposé sur un second côté (231) opposé au premier côté (226) du parcours de transport intégral (220).

6. Dispositif (1) selon une des revendications 2 à 5, **caractérisé en ce que** l'appareil de mesure de lumière transmise (211) présente plus d'une source lumineuse (233, 234, 235, 245, 246), un parcours de rayon principal (236) d'au moins une des sources lumineuses (233, 234, 235; 245, 246) étant disposé orthogonalement à une surface de détection (240) et au moins un autre parcours de rayon principal (236) d'au moins une autre des sources lumineuses (233, 234, 235; 245, 246) étant disposé orienté suivant un angle en divergeant (242) par rapport à une surface de détection (240).

7. Dispositif (1) selon une des revendications 1 à 6, **caractérisé en ce qu'**une unité de détection (72; 210), vue dans le sens axial d'entrée (23) de l'élément bras mobile (20), est disposée axialement en amont d'une unité de détachement (35) permettant de détacher un porte-échantillon (3; 215; 250; 260; 270) de l'élément bras mobile (20).

8. Dispositif (1) selon une des revendications 1 à 6, **caractérisé en ce que** le dispositif de prélèvement (2) comprend une unité de détachement (35) dotée de moyens de raclage (170) permettant de détacher un porte-échantillon utilisé (3; 215; 250; 260; 270) de l'élément bras mobile (20).

9. Dispositif (1) selon une des revendications 1 à 7, **caractérisé par** une unité de détection (72; 210) comportant un capteur thermique pour la détection d'une température des excréments, d'une température du corps, d'un volume des excréments et/ou d'un débit volumétrique d'excréments.

10. Procédé d'utilisation d'un dispositif (1) d'analyse sur place d'excréments, dans lequel un porte-échantillon (3; 215; 250; 260; 270) est déplacé linéairement au moyen d'un élément bras mobile axialement (20) pour un prélèvement d'échantillon par rapport à une unité de détection (72; 210) dans la pièce de guidage (25), dans lequel le porte-échantillon (3; 215; 250; 260; 270) est fixé à un support (30) de l'élément bras mobile axialement (20) et dans lequel un échantillon d'excréments supporté par le porte-échantillon (3; 215; 250; 260; 270) est analysé au moins partiellement dans le dispositif (1), **caractérisé en ce que** la pièce de guidage (25) présente une zone de contamination (132) dotée d'une piste de guidage (26) pour le guidage de l'élément bras mobile (20) et en outre une zone blanche (134) dans laquelle un nouveau porte-échantillon (3; 215; 250; 260; 270) peut être disposé près de la piste de guidage (26) et est transféré par le dispositif de transport (4) vers un support (30) se trouvant sur l'élément bras (20).

11. Procédé selon la revendication 10, **caractérisé en ce qu'**un porte-échantillon utilisé (3; 215; 250; 260; 270) est détaché du support (30) au moyen d'un mouvement axial exécuté dans le sens de déplacement axial (21) de l'élément bras (20).

12. Procédé selon la revendication 10 ou 11, **caractérisé en ce qu'**un porte-échantillon (3; 215; 250; 260; 270) pourvu d'excréments est amené entre un dispositif d'éclairage (225) et un dispositif de détection (230) et est éclairé par transparence pour une mesure de lumière transmise au moyen du dispositif d'éclairage (225) .

13. Procédé selon la revendication 12, **caractérisé en ce que** le porte-échantillon (3; 215; 250; 260; 270) pourvu d'excréments est au repos ou déplacé pendant la mesure lumière transmise.

14. Procédé selon la revendication 12 ou 13, **caractérisé en ce qu'**une aspersion du porte-échantillon (3; 215; 250; 260; 270) avec des excréments, en particulier avec de l'urine, est assistée au moyen de mouvements supplémentaires, en particulier dans le sens de sortie et/ou d'entrée axial (22, 23) de l'élément bras mobile axialement (20).

15. Procédé selon une des revendications 10 à 14, **caractérisé en ce que** les excréments excédentaires, en particulier l'urine excédentaire, sont éliminés, par vibration de l'élément bras mobile axialement (20), de cet élément bras mobile (20) du porte-échantillon (3; 215; 250; 260; 270) et/ou du support (20).

16. Dispositif (14) composé de toilettes (13), d'un urinoir ou similaire et d'un dispositif (1) selon une des revendications 1 à 9.

17. Dispositif selon une des revendications 1 à 9, **caractérisé en ce que** le porte-échantillon (3; 215; 250; 260; 270), pour une analyse sur place des excréments, est pourvu d'un corps de base (216; 251; 271), le porte-échantillon (3; 215; 250; 260; 270) présentant des champs individuels d'analyse d'échantillon (217; 249; 265; 279) de formes géométriques différentes.

18. Dispositif selon la revendication 17, **caractérisé en ce que**, dans le porte échantillon (3; 215; 250 ; 260; 270), aussi bien sur la face avant (252; 262; 272) que sur la face arrière (253; 273) du corps de base (216; 251; 271), des champs d'analyse d'échantillon (217; 249; 265; 279) sont disposés.

19. Dispositif selon une des revendications 17 ou 18, **caractérisé en ce que**, dans le porte échantillon (3; 215; 250; 260; 270), des champs individuels d'analyse d'échantillon (217; 249; 265; 279) présentent des indicateurs différents (218, 219).

20. Dispositif selon une des revendications 17 à 19, **caractérisé en ce que**, dans le porte échantillon (3; 215; 250; 260; 270), des champs individuels d'analyse d'échantillon (217; 249; 265; 279) sont disposés 32 les uns des autres au niveau du corps de base (216; 251; 271).

21. Dispositif selon une des revendications 17 à 20, **caractérisé en ce que**, dans le porte échantillon (3; 215; 250; 260; 270), le corps de base (216; 251; 271) et des champs individuels d'analyse d'échantillon (217; 249; 265; 279) laissent au moins partiellement passer la lumière.
